# EUROPÄISCHE PATENTSCHRIFT

(11) **EP 3 029 055 B1**
(45) Veröffentlichungstag und Bekanntmachung des Hinweises auf die Patenterteilung: **15.03.2017**
(21) Anmeldenummer: 14196197.9
(22) Anmeldetag: 04.12.2014
(51) Int. Cl.: C07F 9/6574, C07B 41/06, C07C 45/50, C07F 15/00

(54) **Monophosphite die einen unsymmetrischen Biaryl-Baustein aufweisen**
Monophosphites with asymmetric biaryl component
Monophosphites présentant un composant en bi-aryle asymétrique

(43) Veröffentlichungstag der Anmeldung: 08.06.2016
(60) Teilanmeldung: 16177738.8
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Selent, Detlef, 18059 Rostock (DE); Börner, Armin, 18059 Rostock (DE)

(56) Entgegenhaltungen:
- WO-A1-85/03702
- WO-A1-2014/056733
- WO-A2-2004/076464
- PETER HANNEN ET AL: "New monodentate chiral phosphite ligands for asymmetric hydrogenation", CHEMICAL COMMUNICATIONS, Nr. 17, 22. Juli 2003 (2003-07-22), Seite 2210, XP055191367, ISSN: 1359-7345, DOI: 10.1039/b306793a

## Beschreibung

Die Erfindung betrifft Monophosphite, die einen unsymmetrischen Biaryl-Baustein aufweisen. Des Weiteren deren Verwendung als Liganden in der Hydroformylierung.

Die erfindungsgemäßen Biaryl-Bausteine weisen beispielsweise eine Phenyl-Phenyl-Einheit, oder eine Naphthyl-Phenyl-Einheit auf.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001 803

WO2014/056733 offenbart asymmetrische Biphosphit-Liganden, deren Verwendung als Ligand in Metall-Komplexen sowie die Verwendung solcher Komplexe zur Katalyse der Hydroformylierung. Peter Hannel et al offenbart in Chem Comm 2003, 17(1), 2210 Monophosphite mit unsymmetrischem Biaryl-Baustein, deren Verwendung als Ligand in Rh-Komplexen und die Verwendung solcher Komplexe als Katalysator in der asymmetrischen Hydrierung.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

In Angew. Chem. Int. Ed. 2000, 39, No. 9, S.1639-1641 von Börner et al. werden Liganden beschrieben, welche eine P-C- und zwei P-O-Bindungen aufweisen, es handelt sich somit um Phosphonite. Die dort beschriebenen Phosphonite weisen bei einem Einsatz in der Hydroformylierung n/iso-Selektivitäten (n/iso = das Verhältnis von linearem Aldehyd (= n) zu verzweigtem (= iso) Aldehyd)) von 0,61 bis 1,57 auf.

Die in DE 199 54 721 beschriebenen Phosphonitliganden weisen eine gute n/iso-Selektivität auf. Allerdings haben eigene Untersuchungen ergeben, dass die Verbindung II-c (in DE 199 54 721; Seite 6) zu einer photochemisch induzierten Zersetzung neigt, weshalb von einem großtechnischen Einsatz abzusehen ist.

Ein Nachteil der Liganden mit einer Phosphonitstruktur besteht in der sehr aufwändigen Herstellung. Die Möglichkeit einer günstigen und einfachen Synthese spielt für den Einsatz von Liganden in einem großtechnischen Prozess aber eine elementare Rolle.

Die einfache Verfügbarkeit und damit verbunden die gute Möglichkeit eines großtechnischen Einsatzes ist ein wichtiges Kriterium, da der Herstellungsaufwand und damit verbunden die anfallenden Kosten nur so hoch sein dürfen, dass die Rentabilität des Gesamtprozesses weiterhin gewährleistet ist.

Rhodium-Monophosphit-Komplexe in katalytisch aktiven Zusammensetzungen sind geeignet für die Hydroformylierung von verzweigten Olefinen mit innenständigen Doppelbindungen. Seit den 1970er Jahren ist die Verwendung von so genannten "bulky phosphites" in der Hydroformylierungbeschrieben (siehe u.a. van Leeuwen et. al, Journal of Catalysis, 2013, 298, 198-205). Diese zeichnen sich durch eine gute Aktivität aus, jedoch ist die n/i-Selektivität für endständig oxierte Verbindungen gering und verbesserungswürdig.

Aus EP 0 155 508 ist die Verwendung von bisarylensubstituierten Monophosphiten bei der rhodiumkatalysierten Hydroformylierung von sterisch gehinderten Olefinen, z. B. Isobuten, bekannt. Hierbei werden jedoch z.T. sehr hohe Rhodiumkonzentrationen verwendet (u.a. 250 ppm), was in Anbetracht des derzeitigen Rhodiumpreises für ein großtechnisches Verfahren inakzeptabel ist und verbessert werden muss.

In der WO 85/03702 A1 wir ein Verfahren zur Hydroformylierung von olefinisch ungesättigten organischen Verbindungen zu Aldehyden beschrieben. Es wird das molare Verhältnis von linearem zu verzweigtem Aldehyd aufgezeigt.

Die WO 2004/076464 A2 offenbart optisch aktive, monodentate Phosphite und Phosphoramidite. Diese weisen jeweils eine axiale Chiralität auf. Die beschriebenen Verbindungen können zur Katalyse von Herstellungsverfahren für optisch aktive Produkte verwendet werden.

Obgleich eine Vielzahl von Liganden und ihre Verwendung in der Rhodium-katalysierten Hydroformylierung bekannt sind, ist es wünschenswert, neue Liganden mit verbesserten Eigenschaften zu entwickeln.

Der Erfindung lag die Aufgabe zugrunde, Monophosphite bereitzustellen, welche gegenüber den bekannten Monophosphiten vorteilhafte Eigenschaften in der Hydroformylierung aufweisen. Ein Beispiel für eine solche Eigenschaft ist eine gesteigerte Ausbeute.
Insbesondere bestand die Aufgabe darin, neue Liganden bereitzustellen, deren Einsatz gegenüber strukturverwandten Monophosphiten, welche ebenfalls einen Biaryl-Baustein aufweisen, zu einer verbesserten Ausbeute führen. Die verbesserte Ausbeute sollte bei zumindest einem Olefin realisiert werden.

Die Aufgabe wird gelöst durch eine Verbindung nach Anspruch 1.

Verbindung, welche die allgemeine Struktur I aufweist: 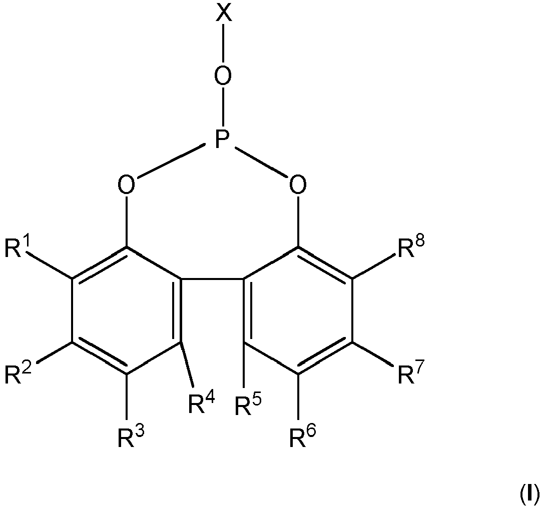 wobei
R¹, R², R³, R⁴, R⁵, R⁶, R⁷, R⁸ ausgewählt sind aus:
   - H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl,, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
X ausgewählt ist aus:
   -(C₁-C₁₂)-Alkyl, -(C₆-C₂₀)-Aryl, -(C₃-C₁₂)-Cycloalkyl, -(C₃-C₁₂)-Heterocycloalkyl,
   und die beiden Reste mindestens eines der vier folgenden Restepaare nicht für den gleichen Rest stehen: R¹ und R⁸, R² und R⁷, R³ und R⁶, R⁴ und R⁵;
   und die Reste R³ und R⁴ sind nicht über eine Kohlenstoffkette miteinander verknüpft;
      wobei die genannten Alkyl- und Arylgruppen wie folgt substituiert sein können:
      substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen, die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
      substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen, diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, - (C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; - SO₃Na, -NO₂, -CN, -NH₂, -N[(C₁-C₁₂)-Alkyl]₂.

Durch das Merkmal "und die beiden Reste mindestens eines der vier folgenden Restepaare nicht für den gleichen Rest stehen: R¹ und R⁸, R² und R⁷, R³ und R⁶, R⁴ und R⁵" wird zum Ausdruck gebracht, dass es sich hierbei um ein unsymmetrisches Biaryl handelt. Die beiden Aromaten lassen sich nicht durch eine zwischen ihnen liegende Spiegelebene aufeinander spiegeln.

Zugelassen sind folgende Restepaarungen, wie beispielsweise:
R¹ ungleich R⁸, R² gleich R⁷, R³ gleich R⁶, R⁴ gleich R⁵;
R¹ gleich R⁸, R² gleich R⁷, R³ ungleich R⁶, R⁴ gleich R⁵.

Ober aber auch Restepaarungen bei denen mehr als nur ein Paar ungleich ist, wie beispielsweise:
R¹ ungleich R⁸, R² gleich R⁷, R³ ungleich R⁶, R⁴ gleich R⁵;
R¹ ungleich R⁸, R² ungleich R⁷, R³ ungleich R⁶, R⁴ gleich R⁵.

Ausgeschlossen wird lediglich der Fall, bei dem alle vier Restepaare jeweils paarweise für den gleichen Rest stehen:
R¹ gleich R⁸, R² gleich R⁷, R³ gleich R⁶, R⁴ gleich R⁵.
Hierbei würde es sich um ein symmetrisches Biaryl handeln.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl, Menthyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte - (C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵, R⁶, R⁷, R⁸ ausgewählt aus:
- H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform ist X ausgewählt aus:
- (C₁-C₁₂)-Alkyl, -(C₆-C₂₀)-Aryl, -(C₃-C₁₂)-Cycloalkyl.

In einer Ausführungsform stehen R¹ und R⁸ nicht für den gleichen Rest.

In einer Ausführungsform stehen R² und R⁷ nicht für den gleichen Rest.

In einer Ausführungsform stehen R³ und R⁶ nicht für den gleichen Rest.

In einer Ausführungsform stehen R⁴ und R⁵ nicht für den gleichen Rest.

In einer Ausführungsform steht X für den folgenden Rest:

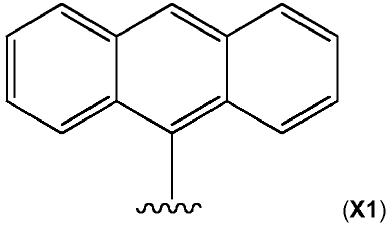

In einer Ausführungsform steht X für den folgenden Rest:

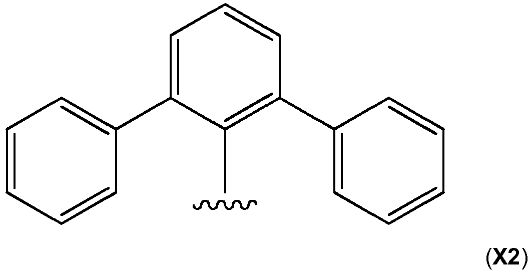

In einer Ausführungsform steht X für den folgenden Rest:

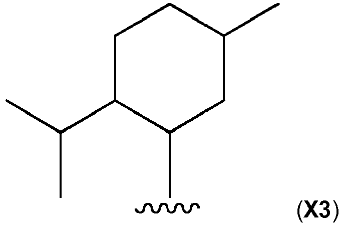

In einer Ausführungsform weist die Verbindung die Struktur einer der Formeln (**4**) bis (**18**) auf:

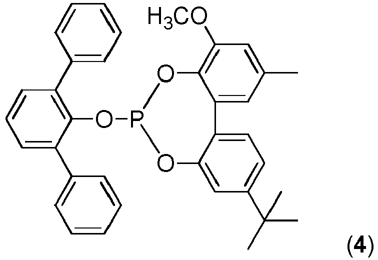

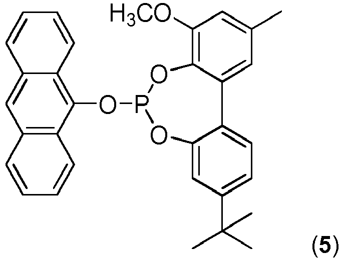

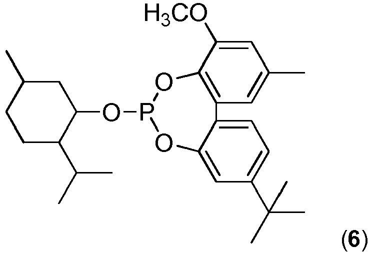

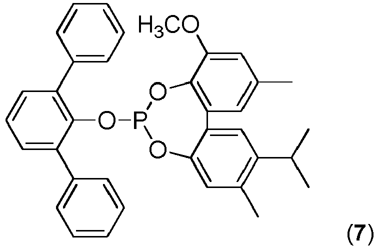

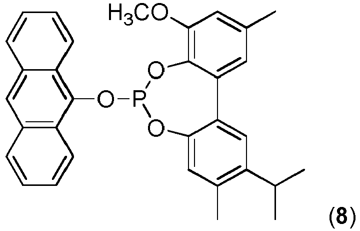

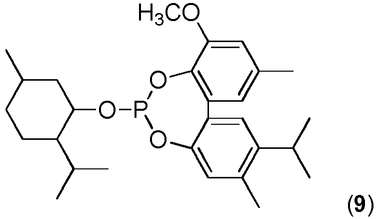

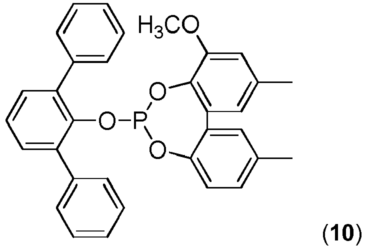

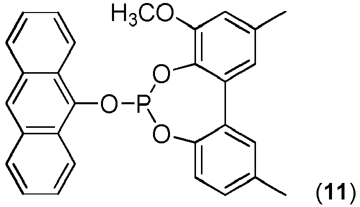

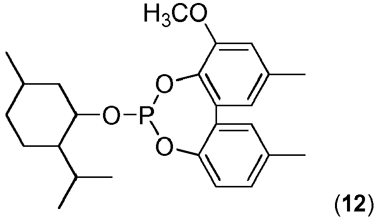

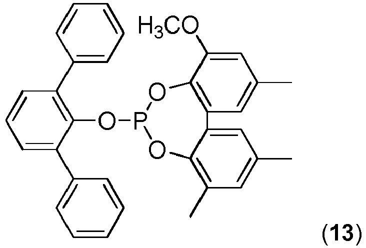

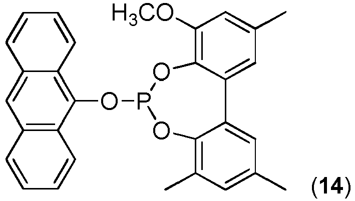

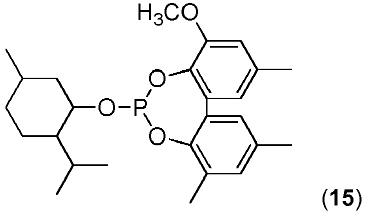

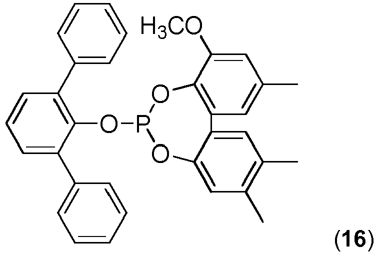

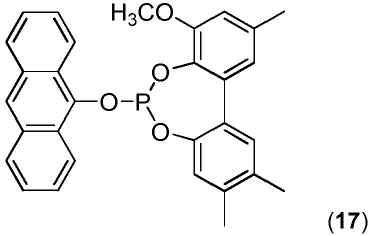

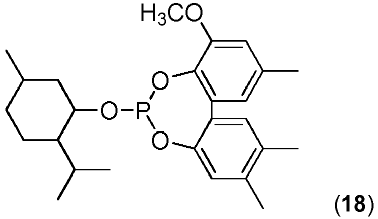

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

Es kann hierbei auch ein Überschuss an Liganden verwendet werden und nicht zwangläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2-oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen und einer Figur näher erläutert.

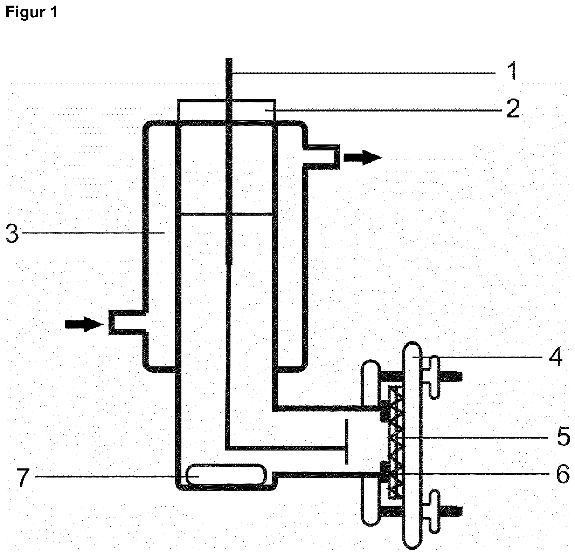

Die Figur 1 zeigt eine Reaktionsapparatur, in welcher die Kupplungsreaktion zu den entsprechenden unsymmetrischen Biarylen durchgeführt werden kann. Die Apparatur umfasst eine Nickelkathode (1) und eine Anode aus Bor-dotiertem Diamant (BDD) auf Silizium (5). Die Apparatur kann mit Hilfe des Kühlmantels (3) gekühlt werden. Die Pfeile deuten hierbei die Durchflussrichtung des Kühlwassers an. Der Reaktionsraum ist mit einem Teflonstopfen (2) verschlossen. Das Reaktionsgemisch wird durch ein Magnetrührstäbchen (7) durchmischt. Auf der anodischen Seite wird die Apparatur durch Schraubzwingen (4) und Dichtungen (6) verschlossen.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Synthese der unsymmetrischen Biaryle

Die unsymmetrischen Biaryle wurden durch ein elektrochemisches Verfahren durch Kupplung von zwei Phenolen bzw. von einem Naphthol mit einem Phenol, welche sich in ihrem Oxidationspotential unterscheiden, hergestellt. Siehe hierzu auch B. Elsler, D. Schollmeyer, K. M. Dyballa, R. Franke, S. R. Waldvogel, "Metall- und reagensfreie hochselektive anodische Kreuzkupplung von Phenolen", Angew. Chem., 2014, DOI: 10.1002/ange.201400627

### Allgemeine Arbeitsvorschrift:

Die Kupplungsreaktion wurde in einer Apparatur durchgeführt, wie sie in Figur 1 dargestellt ist. 5 mmol des ersten Phenols mit einem Oxidationspotential *E_{Ox}1* werden mit 15 mmol des zweiten Phenols mit einem Oxidationspotential *E_{Ox}2* in den in der nachfolgenden Tabelle 1 angegebenen Mengen in 1,1,1,3,3,3-Hexafluorisopropanol (HFIP) und MeOH oder in Ameisensäure und MeOH gelöst. Die Elektrolyse erfolgt galvanostatisch. Der Außenmantel der Elektrolysezelle wird über einen Thermostaten auf etwa 10 °C temperiert, während die Reaktionsmischung gerührt und auf 50 °C mit Hilfe eines Sandbades erhitzt wird. Nach Ende der Elektrolyse wird der Zellinhalt mit Toluol in einen 50 mL Rundhalskolben überführt und das Lösungsmittel unter vermindertem Druck am Rotationsverdampfer bei 50 °C, 200-70 mbar entfernt. Nicht umgesetztes Edukt wird mittels Kurzwegdestillation zurückerhalten (100 °C, 10⁻³ mbar).

**Elektrodenmaterial**

| | |
|---|---|
| Anode: | Bor-dotierter Diamant (BDD) auf Si |
| Kathode: | Ni-Netz |

**Elektrolysebedingungen:**

| | |
|---|---|
| Temperatur [T]: | 50 °C |
| Stromstärke [I]: | 15 mA |
| Stromdichte [j]: | 2.8 mA/cm² |
| Ladungsmenge [Q]: | 2 F/mol Unterschusskomponente |
| Klemmspannung [Uₘₐₓ]: | 3-5 V |

Die Synthese der Biaryle erfolgte gemäß der oben beschriebenen allgemeinen Arbeitsvorschrift, und in einer Reaktionsapparatur, wie sie in Figur 1 dargestellt ist.

### 2,2'-Dihydroxy-4',5-dimethyl-5'-(methylethyl)-3-methoxybiphenyl

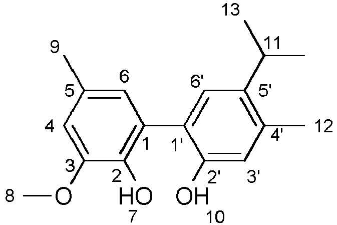

Es wurden 0.69 g (5 mmol, 1.0 Äquiv.) 4-Methylguajacol und 2.28 g (15 mmol, 3.0 Äquiv.) 3-Methyl-4-(methylethyl)phenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten. Ausbeute: 716 mg (50%, 2.5 mmol)
GC (Methode *hart,* HP-5): t_{R}= 14.87 min
R_{f}(CH:EE= 4:1)= 0.43
mₚ= 126.8°C (aus CH umkristallisiert)
¹H-NMR (600 MHz, DMSO) δ= 1.17-1.12 (m, 6H, 13-H), 2.24 (m, 6H, 9-H/12-H), 3.01 (dt, 1H, 11-H), 3.79 (s, 3H, 8-H), 6.55 (s, 1 H, 6-H), 6.66 (d, 1 H, 6'-H), 6.73 (d, 1 H, 4-H), 6.96 (s, 1 H, 3'-H), 8.16 (s, 1H, 7-H), 8.84 (s, 1H, 10-H);
Kopplungen: ⁴J_{4-H, 6-H}= 2.2 Hz, ⁴J_{6'-H, 11-H}= 2.9 Hz, ³J_{11-H, 13-H}= 6.8 Hz;
¹³C-NMR (151 MHz, DMSO) δ= 18.73, 20.80 (C-9/C-12), 23.54 (C-13), 28.10 (C-11), 55.78 (C-8), 111.23 (C-4), 117.34 (C-6'), 123.42 (C-1'), 123.49 (C-6), 126.43 (C-1), 127.36 (C-5), 127.49 (C-3'), 134.40 (C-5'), 136.62 (C-4'), 141.12 (C-2), 147.65 (C-3), 151.69 (C-2').
HRMS für C₁₈H₂₂O₃ (ESI+) [M+Na⁺]: ber: 309.1467, gef.: 309.1457
MS (EI, GCMS): m/z(%): 286 (50) [M]⁺˙, 271 (100) [M-CH₃˙]⁺, 244 (22) [M-C₃H₆˙]⁺.
Elementaranalyse für C₁₈H₂₂O_{3:} ber: C: 75.50%, H: 7.74%, gef.: C: 75.01%, H: 7.70%.

### 2,2'-Dihydroxy-5,5'-dimethyl-3-methoxybiphenyl

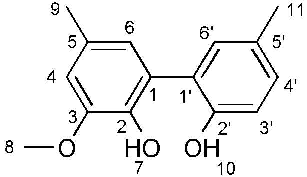

Es wurden 1.66 g (12 mmol, 1.0 Äquiv.) 4-Methylguajacol und 3.91 g (36 mmol, 3.0 Äquiv.) 4-Methylphenol in 65 mL HFIP und 14 mL MeOH gelöst, 1.63 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 440 mg (36%, 1.8 mmol)
GC (Methode *hart,* HP-5): t_{R}= 13.56 min
R_{f}(CH:EE= 4:1)= 0.38
mₚ= 162.0 °C (aus CH umkristallisiert)
¹H-NMR (400 MHz, DMSO) δ= 2.18 (s, 3H, 9-H/11-H), 2.21 (s, 3H, 9-H/11-H), 3.76 (s, 3H, 8-H), 6.53 (s, 1 H, 6-H), 6.71 (s, 1 H, 4-H), 6.75 (d, 1 H, 3'-H), 6.86-6.94 (m, 2H, 4'-H/6'-H), 8.53 (bs, 1 H, 7-H/12-H);
Kopplungen: ³J_{3'-H, 4'-H}= 8.4 Hz;
¹³C-NMR (101 MHz, DMSO) δ= 20.21, 20.77 (C-9/C-11), 55.79 (C-8), 111.36 (C-4), 115.69 (C-3'), 123.50 (C-6), 125.72 (C-1'), 126.16 (C-1), 127.20 (C-5), 127.30 (C-5'), 128.50 (C-6'), 131.83 (C-4'), 141.20 (C-2), 147.61 (C-3), 152.11 (C-2').
HRMS für C₁₅H₁₆O₃ (ESI+) [M+Na⁺]: ber: 267.0997, gef.: 267.0999
MS (EI, GCMS): m/z(%): 244 (100) [M]⁺˙, 229 (64) [M-CH₃˙]⁺.
Elementaranalyse für C₁₅H₁₆O_{3:} ber: C: 73.75%, H: 6.60%, gef.: C: 73.81%, H: 6.54%.

### 2,2'-Dihydroxy-3-methoxy-3',5,5'-trimethyl-biphenyl

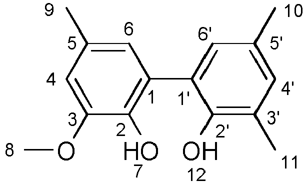

Es wurden 0.70 g (6 mmol, 1.0 Äquiv.) 4-Methylguajacol und 2.08 g (17 mmol, 3.0 Äquiv.) 2,4-Dimethylphenol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als leichtgelber Feststoff erhalten.
Ausbeute: 663 mg (45%, 2.5 mmol)
GC (Methode *hart,* HP-5): t_{R}= 13.97 min
R_{f}(CH:EE= 4:1)= 0.29
mₚ= 119.7 °C (aus DCM/CH umkristallisiert)
¹H-NMR (400 MHz, CDCl₃) δ= 2.34 (s, 3H, 10-H), 2.35 (s, 3H, 11-H), 2.38 (s, 3H, 9-H), 3.94 (s, 3H, 8-H), 6.16 (s, 1H, 12-H), 6.20 (s, 1H, 7-H), 6.76 (d, 1H, 4-H), 6.78 (d, 1H, 6-H), 6.98 (d, 1H, 6'-H), 7.03 (d, 1H, 4'-H);
Kopplungen: ⁴J_{4-H, 6-H}= 1.7 Hz, ⁴J_{4'-H, 6'-H}= 2.1 Hz;
¹³C-NMR (101 MHz, CDCl₃) δ= 16.51 (C-9), 20.54 (C-10), 21.20 (C-11), 56.12 (C-8), 110.92 (C-4), 123.95 (C-6), 124.13 (C-1), 124.64 (C-1'), 126.18 (C-3'), 128.82 (C-6'), 129.59 (C-5'), 130.40 (C-5), 131.40 (C-4'), 139.46 (C-2), 146.35 (C-3), 149.42 (C-2').
HRMS für C₁₈H₁₆O₃ (ESI+) [M+Na⁺]: ber: 281.1154, gef.: 281.1152
MS (EI, GCMS): m/z(%): 242 (100) [M]⁺˙, 227 (38) [M-CH₃˙]⁺.
Elementaranalyse für C₁₆H₁₈O_{3:} ber: C: 68.31%, H: 6.45%, gef.: C: 68.29%, H: 6.40%.

### 2,2'-Dihydroxy-3-methoxy-5-methyl-4'-(dimethylethyl)biphenyl

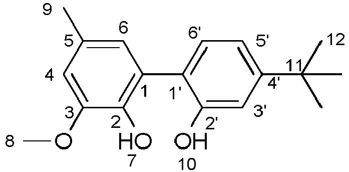

Es wurden 0.69 g (5 mmol, 1.0 Äquiv.) 4-Methylguajacol und 2.25 g (15 mmol, 3.0 Äquiv.) 3-tert-Butylphenol in 33 mL HFIP gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 808 mg (63%, 3.1 mmol)
GC (Methode *hart,* HP-5): t_{R}= 13.97 min
R_{f}(CH:EE= 4:1)= 0.29
mₚ= 160.3 °C (aus DCM/CH umkristallisiert)
¹H-NMR (400 MHz, CDCl₃) δ= 1.37 (s, 9H, 12-H), 2.36 (s, 3H, 9-H), 3.94 (s, 3H, 8-H), 6.25 (s, 1 H, 7-H), 6.48 (s, 1 H, 10-H), 6.75 (d, 1 H, 6-H), 6.79 (d, 1 H, 4-H), 7.08 (dd, 1 H, 5'-H), 7.12 (d, 1 H, 3'-H), 7.27 (d, 1 H, 6'-H);
Kopplungen: ⁴J_{4-H, 6-H}= 1.7 Hz; ³J_{5'-H, 6'-H}= 8.0 Hz, ⁴J_{3'-H}, _{5'-H}= 1.7 Hz;
¹³C-NMR (101 MHz, CDCl₃) δ= 21.24 (C-9), 31.31 (C-12), 34.58 (C-11), 56.15 (C-8), 110.79 (C-4), 114.94 (C-3'), 118.30 (C-5'), 122.37 (C-1'), 123.88 (C-1), 123.94 (C-6), 130.45 (C-6'), 130.53 (C-4'), 139.24 (C-5), 146.32 (C-3), 152.91 (C-2'), 153.13 (C-2).
HRMS für C₁₅H₁₆O₄ (ESI+) [M+Na⁺]: ber: 309.1467, gef.: 309.1466
MS (EI, GCMS): m/z(%): 242 (100) [M]⁺˙, 227 (38) [M-CH₃˙]⁺.
Elementaranalyse für C₁₈H₂₂O_{3:} ber: 75.50%, H: 7.74%, gef.: C: 75.41%, H: 7.72%.

### 2,2'-Dihydroxy-4',5-dimethy-3-methoxylbiphenyl

Es wurden 0.70 g (5 mmol, 1.0 Äquiv.) 4-Methylguajacol und 1.65 g (15 mmol, 3.0 Äquiv.) 3-Methylphenol in 33 mL HFIP gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und zwei Kreuzkupplungsprodukte als farblose Feststoffe erhalten.

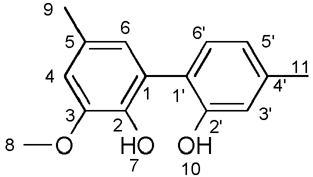

Ausbeute: 266 mg (21%, 1.1 mmol)
GC (Methode *hart,* HP-5): t_{R}= 13.72 min
R_{f}(CH:EE= 4:1)= 0.25
mₚ= 136.2 °C (aus DCM/CH umkristallisiert)
¹H-NMR (400 MHz, CDCl₃) δ= 2.35 (s, 3H, 9-H/11-H), 2.37 (s, 3H, 9-H/11-H), 3.94 (s, 3H, 8-H), 6.17 (s, 1 H, 10-H), 6.35 (s, 1H, 2-H), 6.74 (d, 1H, 4-H), 6.76 (s, 1 H, 6-H), 6.88-6.83 (m, 1H, 5'-H), 6.90 (d, 1 H, 3'-H), 7.21 (d, 1 H, 6'-H);
Kopplungen: ⁴J_{4-H, 6-H}= 1.8 Hz, ³J_{5'-H, 6'-H}= 7.7 Hz, ⁴J_{3'-H}, _{5'-H}= 1.5 Hz;
¹³C-NMR (101 MHz, CDCl₃) δ= 21.11, 21.20 (C-9/C-11), 56.13 (C-8), 110.81 (C-4), 118.25 (C-3'), 121.97 (C-5'), 122.39 (C-1), 123.77 (C-1'), 123.85 (C-6), 130.50 (C-5), 130.68 (C-6'), 139.30 (C-4'), 139.54 (C-2), 146.31 (C-3), 153.33 (C-2').
HRMS für C₁₅H₁₆O₃ (ESI+) [M+Na⁺]: ber: 267.0997, gef.: 267.1006
MS (EI, GCMS): m/z(%): 244 (100) [M]⁺˙, 229 (18) [M-CH₃˙]^{+,}.
Elementaranalyse für C₁₅H₁₆O_{3:} ber: C: 73.75%, H: 6.60%, gef.: C: 73.70%, H: 6.68%.

### 2,2'-Dihydroxy-3-methoxy-4'-5,5'-trimethylbiphenyl

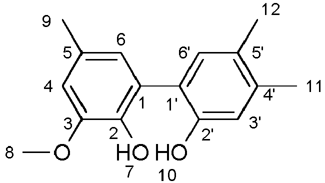

Es wurden 0.69 g (5 mmol, 1.0 Äquiv.) 4-Methylguajacol und 1.83 g (15 mmol, 3.0 Äquiv.) 3,4-Dimethylphenol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 688 mg (52%, 2.6 mmol)
GC (Methode *hart,* HP-5): t_{R}= 14.52 min
R_{f}(CH:EE= 4:1)= 0.29
mₚ= 152.3 °C (aus DCM/CH umkristallisiert)
¹H-NMR (400 MHz, CDCl₃) δ= 12.25 (s, 3H, 11-H), 2.28 (s, 3H, 12-H), 2.36 (s, 3H, 9-H), 3.93 (s, 3H, 8-H), 6.19 (s, 1 H, 7-H), 6.25 (s, 1 H, 10-H), 6.73 (d, 1 H, 4-H), 6.76 (s, 1 H, 6-H), 6.88 (s, 1 H, 3'-H), 7.08 (s, 1H, 6'-H);
Kopplungen: ⁴J_{4-H, 6-H}= 1.7 Hz;
¹³C-NMR (101 MHz, CDCl₃) δ= 18.89 (C-11), 19.60 (C-12), 21.24 (C-9), 56.14 (C-8), 110.74 (C-4), 118.93 (C-3'), 122.54 (C-1), 123.82 (C-6), 123.97 (C-1'), 129.03 (C-5), 130.46 (C-4'), 131.69 (C-6'), 137.94 (C-5'), 139.26 (C-2), 146.31 (C-3), 151.36 (C-2').
HRMS für C₁₆H₁₈O₃ (ESI+) [M+Na⁺]: ber: 281.1154, gef.: 281.1157
MS (EI, GCMS): m/z(%): 258 (100) [M]⁺˙, 243 (10) [M-CH₃˙]⁺.
Elementaranalyse für C₁₆H₁₈O_{3:} ber: 74.39%, H: 7.02%, gef.: C: 74.32%, H: 7.20%

### 2,2'-Dihydroxy-5'-isopropyl-3-methoxy-5-methylbiphenyl

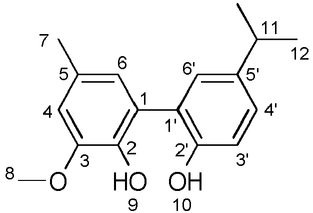

Es wurden 0.69 g (5 mmol, 1.0 Äquiv.) 4-Methylguajacol und 2.05 g (15 mmol, 3.0 Äquiv.) 4-Isopropylphenol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 0.53 g (39%, 1.9 mmol)
GC (Methode *hart,* HP-5): t_{R}= 14.23 min
R_{f}(CH:EE= 4:1)= 0.30
¹H-NMR (400 MHz, CDCl₃) δ= 1.27 (m, 6H), 2.36 (s, 3H), 2.91 (dt, J= 13.8, 6.9, 6.9 Hz, 1H), 3.94 (s, 3H), 6.13-6.27 (m, 2H), 6.82-6.65 (m, 1H), 6.25 (m, 2H), 6.75 (s, 1H), 6.77 (s, 1H), 6.99 (d, J= 8.1 Hz, 1 H), 7.19-7.12 (m, 2H);
¹³C-NMR (101 MHz, CDCl₃) δ= 21.25, 24.27, 33.40, 56.18, 110.92, 117.60, 123.91, 124.23, 125.07, 127.29, 128.80, 130.57, 139.29, 141.42, 146.31, 151.51.
HRMS für C₁₇H₂₀O₃ (ESI+) [M+Na⁺]: ber: 295.1310, gef.: 295.1297
MS (EI, GCMS): m/z(%): 272 (80) [M]⁺˙, 257 (100) [M-CH₃˙]⁺.

### 2,2'-Dihydroxy-3-methoxy-5-methyl-5'-tert-butylbiphenyl

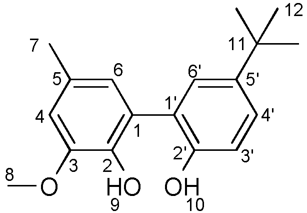

Es wurden 0.69 g (5 mmol, 1.0 Äquiv.) 4-Methylguajacol und 2.26 g (15 mmol, 3.0 Äquiv.) *4-Tert-*butylphenol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.48 g (34%, 1.7 mmol)
GC (Methode *hart,* HP-5): t_{R}= 14.52 min
R_{f}(CH:EE= 4:1)= 0.24
¹H-NMR (400 MHz, CDCl₃) δ= 1.34 (s, 9H), 2.37 (s, 3H), 3.94 (s, 3H), 6.17 (s, 1H), 6.24 (s, 1H), 6.75 (s, 1 H), 6.77 (s, 1 H), 6.99 (d, J= 8.4 Hz, 1 H), 7.31-7.29 (m, 1 H), 7.33 (dd, J= 8.4, 2.5 Hz, 1 H).
¹³C-NMR (101 MHz, CDCl₃) δ= 21.28, 31.61, 34.20, 56.18, 110.91, 117.25, 123.92, 124.41, 124.63, 126.38, 127.78, 130.58, 139.32, 143.70, 146.32, 151.22.
HRMS für C₁₈H₂₂O₃ (ESI+) [M+Na⁺]: ber: 309.1467, gef.: 309.1476
MS (EI, GCMS): m/z(%): 286 (28) [M]⁺˙, 271 (100) [M-CH₃˙]⁺.

### 2,2'-Dihydroxy-3',5'-di-tert-butyl-5-methyl-3-methoxybiphenyl

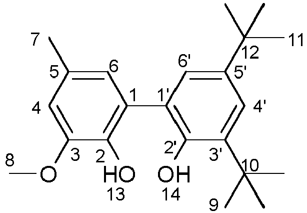

Es wurden 0.69 g (5 mmol, 1.0 Äquiv.) 4-Methylguajacol und 3.12 g (15 mmol, 3.0 Äquiv.) 2,4-Di*tert*-butylphenol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 0.41 g (24%, 1.2 mmol)
GC (Methode*hart*, HP-5): t_{R}= 15.15 min
R_{f}(CH:EE= 9:1)= 0.35
mₚ= 120.2 °C (inn-Pentan umkristallisiert)
¹H-NMR (400 MHz, CDCl₃) δ= 1.36 (s, 9H), 1.50 (s, 9H), 2.38 (s, 3H), 3.96 (s, 3H), 6.00 (s, 1H), 6.05 (s, 1H), 6.77 (s, 1 H), 7.16 (d, J= 2.5 Hz, 1 H), 7.39 (d, J= 2.5 Hz, 1 H).
¹³C-NMR (101 MHz, CDCl₃) δ= 21.23, 29.88, 31.69, 34.40, 35.23, 56.17, 111.03, 123.96, 124.17, 125.09, 125.50, 130.42, 136.73, 139.72, 142.36, 146.45, 149.82.
MS (EI, GCMS): m/z(%): 342 (22) [M]⁺˙, 327 (100) [M-CH₃˙]⁺.

### 2,2'-Dihydroxy-3',5-dimethyl-3-methoxy-5'-tert-butylbiphenyl

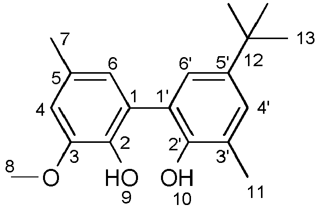

Es wurden 0.69 g (5 mmol, 1.0 Äquiv.) 4-Methylguajacol und 2.47 g (15 mol, 3.0 Äquiv.) 2-Methyl-4-*tert*-butylphenol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.69 g (46%, 2.3 mmol)
GC (Methode *hart,* HP-5): t_{R}= 14.79 min
R_{f}(CH:EE= 4:1)= 0.33
¹H-NMR (400 MHz, CDCl₃) δ= 1.37 (s, 9H), 2.39 (d, J= 2.4 Hz, 6H), 3.94 (s, 3H), 6.15 (s, 1H), 6.17 (s, 1H), 6.77 (s, 1H), 6.79 (s, 1 H), 7.17 (d, J= 2.5 Hz, 1 H), 7.24 (d, J= 2.4 Hz, 1 H); ¹³C-NMR (101 MHz, CDCl₃) δ= 16.90, 21.28, 31.67, 34.12, 56.16, 110.94, 124.02, 124.17, 124.59, 125.41, 125.65, 127.86, 130.47, 139.50, 143.07, 146.40, 149.41.
MS (EI, GCMS): m/z(%): 300 (18) [M]⁺˙, 285 (100) [M-CH₃˙]⁺.

### 2,2'-Dihydroxy-3-methoxy-5-methyl-5'-(1-methylethyl)biphenyl

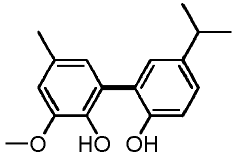

Es wurden 0.69 g (5 mmol, 1.0 eq.) 4-Methylguajacol und 2.05 g (15 mmol, 3.0 eq.) 4-isopropylphenol und 0.68 g MTES in 27 mL HFIP + 6 mL MeOH MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 39%, 527 mg, 1.9 mmol.
R_{f} (Cyclohexane:Ethylacetat = 4:1)= 0.30;
¹H NMR (400 MHz, CDCl₃) δ= 1.27 (m, 6H), 2.36 (s, 3H), 2.91 (sept, J= 6.9 Hz, 1 H), 3.94 (s, 3H), 6.13-6.27 (m, 2H), 6.65-6.82 (m, 2H), 6.99 (d, *J=* 8.1 Hz, 1 H), 7.12-7.19 (m, 2H);
¹³C NMR (101 MHz, CDCl₃) δ= 21.37, 24.39, 33.53, 56.31, 111.04, 117.73, 124.04, 124.36, 125.20, 127.42, 128.93, 130.70, 139.42, 141.55, 146.44, 151.64.
HRMS für C₁₇H₂₀O₃ (ESI+) [M+Na⁺]: berechnet.: 295.1310, gemessen: 295.1297;
MS (EI, GCMS): m/z(%): 272 (80) [M]⁺˙, 257 (100) [M-CH₃˙]⁺.

### 2,2'-Dihydroxy-3-methoxy-5-methyl-4'-(methylethyl)biphenyl

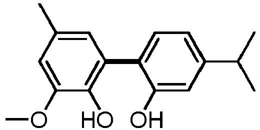

Es wurden 0.69 g (5 mmol, 1.0 eq.) 4-Methylguajacol und 2.065 g (15 mmol, 3.0 eq.) 3-isopropylphenol und 0.68 g MTES in 33 mL HFIP gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten (Ausbeute: 52%, 705 mg, 2.6 mmol).
R_{f} (Cyclohexane:Ethylacetat = 4:1)= 0.29.
¹H NMR (400 MHz, CDCl₃) δ= 1H NMR (400 MHz, CDCl3) δ 1.27 (s, 3H), 1.29 (s, 3H), 2.34 (s, 3H), 2.91 (sept, *J*= 7.0 Hz, 1H), 3.94 (s, 3H), 6.15 (s, 1H), 6.35 (s, 1H), 6.73 (d, *J*= 1.8 Hz, 1H), 6.75-6.77 (m, 1 H), 6.90 (dd, *J*= 7.9 Hz, 1.8 Hz, 1 H), 6.94 (d, *J*= 1.7 Hz, 1 H), 7.23 (d, *J*= 7.8 Hz, 1 H).
¹³C NMR (101 MHz, CDCl₃) δ=¹³C NMR (101 MHz, CDCl₃) δ 21.36, 24.02, 33.92, 56.30, 77.16, 110.91, 115.77, 119.56, 122.81, 124.00, 124.08, 130.65, 130.84, 139.38, 146.43, 150.72, 153.54. HRMS für C₁₇H₂₀O₃ (ESI+) [M+Na⁺]: berechnet: 295.1310, gemessen: 295.1305; MS (EI, GCMS): m/z(%): 272 (100) [M]⁺˙, 257 (50) [M-CH₃˙]. ˙]^{+,}. Elementaranalyse für C₁₇H₂₀O_{3:} berechnet: 74.97%, H: 7.40%, gemessen: C: 75.05%, H: 7.36%.

### 2,2'-Dihydroxy-4',5-dimethyl-3-methoxybiphenyl

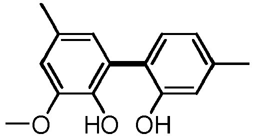

Es wurden 0.28 g (2 mmol, 1.0 eq.) 4-Methylguaiacol, 1.22 g (6 mmol, 3.0 eq.) 3-Methylphenyl und 0.77 g MTBS 25 mL HFIP gelöst und der Elektrolyt in die Beaker-typ Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und führte zu den beiden Kreuzkupplungsprodukten als farbloses und viskoses Öl.
Ausbeute: 21%, 266 mg, 1.1 mmol; R_{f} (Cyclohexane:Ethylacetat = 4:1)= 0.25; mₚ= 136.2 °C (kristallisiert aus Dichloromethan/Cyclohexan);
¹H NMR (400 MHz, CDCl₃) δ= 2.35 (s, 3H), 2.37 (s, 3H,), 3.94 (s, 3H), 6.17 (s, 1 H), 6.35 (s, 1 H), 6.74 (d, *J=* 1.8 Hz, 1 H), 6.76 (s, 1 H), 6.88-6.83 (m, 1 H), 6.90 (d, 1 H, *J=* 1.5 Hz), 7.21 (d, 1 H, *J=* 7.7 Hz);
¹³C NMR (101 MHz, CDCl₃) δ= 21.11, 21.20 56.13, 110.81, 118.25, 121.97, 122.39, 123.77, 123.85, 130.50, 130.68, 139.30, 139.54, 146.31, 153.33.
HRMS für C₁₅H₁₆O₃ (ESI+) [M+Na⁺]: berechnet: 267.0997, gemessen: 267.1006; MS (EI, GCMS): m/z(%): 244 (100) [M]⁺˙, 229 (18) [M-CH₃˙]^{+,}. Elementaranalyse für C₁₅H₁₆O_{3:} berechnet: C: 73.75%, H: 6.60%, gemessen: C: 73.70%, H: 6.68%.

### 2,2'-Dihydroxy-5,5'-dimethyl-3'-(1,1-dimethylethyl)-3-methoxybiphenyl

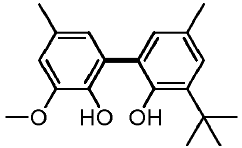

Es wurden 0.69 g (5 mmol, 1.0 eq.) 4-Methylguaiacol, 2.47 g (15 mmol, 3.0 eq.) 4-Methyl-2-*tert-*butylphenol und 0.68 g MTES in 27 mL HFIP + 6 mL MeOH gelöst gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbes Öl erhalten (Ausbeute: 36%, 545 mg, 1.8 mmol).
R_{f} (Cyclohexane:Ethylacetat = 9:1)= 0.36;
¹H NMR (400 MHz, CDCl₃) δ= 1.46 (s, 9H), 2.34 (m, 6H), 3.93 (s, 3H), 5.99 (s, 1 H), 6.01 (s, 1 H), 6.74 (s, 2H), 6.96 (d, *J*= 1.9 Hz, 1 H), 7.14 (d, *J*= 1.9 Hz, 1 H);
¹³C NMR (101 MHz, CDCl₃) δ= 21.05, 21.32, 29.96, 35.05, 56.30, 77.16, 111.21, 124.18, 124.24, 125.92, 127.67, 129.15, 129.22, 130.51, 137.57, 139.87, 146.57, 150.10.
HRMS für C₂₂H₃₀O₃ (ESI+) [M+Na⁺]: berechnet: 323.1623, gemessen: 323.1618; MS (EI, GCMS): m/z(%): 300 (100) [M]⁺˙, 285 (100) [M-CH₃˙]⁺.

### Synthese der Chlorophosphite

2,6-Diphenylphenoxy-dichlorphosphin wurde nach W. Maringgele, A. Meler, Phosphorus, Sulfur and Silicon, 1994, 90, 235-241 hergestellt.

Dichlor((-)-menthyloxy)phosphin wurde nach T. Imamoto, T. Yoshizawa, K. Hirose, Y. Wada, H. Masuda, K. Yamaguchi, H. Seki, Heteroatom Chemistry, 1995, 6, 99-104 hergestellt.

(Anthracen-9-yloxy)dichlorophosphin wurde nach nachfolgender Synthesevorschrift hergestellt:
Zu einer gerührten Lösung von PCl₃ (5,16 g; 37,6 mmol) in THF (25 ml) wird bei 10 °C tropfenweise innerhalb von 90 min eine Mischung aus Anthron (2,03 g; 10,44 mmol) und Triethylamin (2 ml) in THF (80 ml) gegeben. Nach Stehenlassen über Nacht wird filtriert, das Filtrat im Vakuum zur Trockne gebracht und der erhaltene Rückstand in Toluol (50 ml) aufgenommen. Man filtriert erneut, entfernt das Lösungsmittel im Vakuum und trocknet den gelben Rückstand bei 50°C/ 0,1 mbar. Anschließend wird der erhaltene Feststoff mit Hexan (30 ml) bei Raumtemperatur über Nacht verrührt. Man filtriert und wäscht den Filterkuchen mit Hexan (3x20 ml). Ausbeute: 2,27 g (73%). ³¹P-NMR (CD₂Cl₂): d 202,5 (s) ppm.

### Synthese der Liganden

### 6-([1,1':3',1"-Terphenyl]-2'-yloxy)-9-(tert-butyl)-4-methoxy-2-methyldibenzo[d,f][1,3,2]dioxaphosphepin.

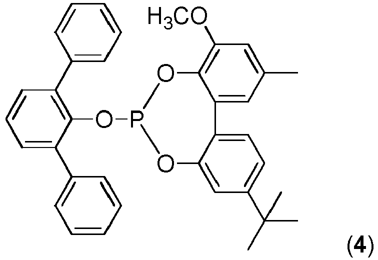

Eine gerührte Lösung von 4'-(*tert*-Butyl)-3-methoxy-5-methyl-[1,1'-biphenyl]-2,2'-diol (0,479 g; 1,67 mmol) in Toluol (12 ml) wurde mit Triethylamin (1,550 g; 15,32 mmol) versetzt und auf 0 °C gekühlt. Eine Lösung von 2,6-Diphenylphenoxydichlorphosphin (0,581 g; 1,673 mmol) in Toluol (3 ml) wurde tropfenweise zugegeben. Die Reaktionsmischung wurde über Nacht gerührt und dann filtriert. Das Filtrat wurde im Vakuum bis zur Trockne eingeengt und der erhaltene Feststoff bei 50°C /0,1 mbar getrocknet. Ausbeute: 0,890 g (1,588 mmol; 96 %). Elementaranalyse (ber. für C₃₆H₃₃O₄P = 560,63 g/ mol) C 77,07 (77,13); H 5,96 (5,93); P 5,62 (5,52) %.
³¹P-NMR (CD₂Cl₂): 144,5 ppm.
¹H-NMR (CD₂Cl₂): 41 (s, 9 H); 3,82 (s, 3 H); 6,81 (m, 3 H, Hₐᵣₒₘ); 7,21-7,64 (m, 15 H, Hₐᵣₒₘ) ppm.
¹³C-NMR (CD₂Cl₂): 21,7; 31,5; 35,0; 56,4; 112,9; 119,3; 121,5; 122,3; 125,0; 125,7; 127,7; 128,4; 128,6; 129,4; 130,7; 131,1; 132,3 (d, *J*_{CP}= 3,8 Hz); 135,5; 136,3 (d, *J*_{CP}= 3,5 Hz); 138,9; 146,9 (d, *J*_{CP}= 4,0 Hz); 149,8 (d, *J*_{CP}= 8,1 Hz); 151,9; 153,1 ppm.

### 6-(Anthracen-9-yloxy)-9-(tert-butyl)-4-methoxy-2-methyldibenzo[d,f][1,3,2]dioxaphosphepin.

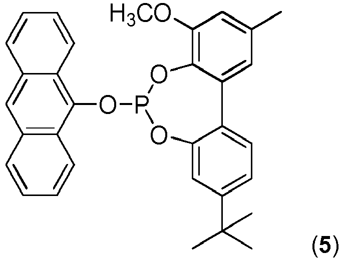

Eine gerührte Suspension von 4'-(*tert*-Butyl)-3-methoxy-5-methyl-[1,1'-biphenyl]-2,2'-diol (0,703 g; 2,46 mmol) in Toluol (7 ml) wurde mit Triethylamin (2,277 g; 22,50 mmol) versetzt und diese Mischung tropfenweise bei 0 °C zu einer Lösung von (Anthracen-9-yloxy)dichlorophosphin (0,725 g; 2,46 mmol) in Toluol (13 ml) gegeben. Die Reaktionsmischung wurde über Nacht gerührt und dann filtriert. Das Filtrat wurde im Vakuum bis zur Trockne eingeengt und der erhaltene Rückstand aus Toluol (2 ml) umkristallisiert. Ausbeute: 0,320 g (0,630 mmol; 26 %).

Elementaranalyse (ber. für C₃₂H₂₉O₄P = 508,55 g/ mol) C 75,48 (75,58); H 5,86 (5,75); P 6,03 (6,09) %.
³¹P-NMR (CD₂Cl₂): 148,4 ppm.
¹H-NMR (CD₂Cl₂): 44 (s, 9 H); 2,49 (m, 3 H); 4,00 (s, 3 H); 6,94-7,04 (m, 2 H, Hₐᵣₒₘ); 7,46 (m, 2 H, Hₐᵣₒₘ); 7,56-7,69 (m, 5 H, Hₐᵣₒₘ); 8,10 (m, 2 H, Hₐᵣₒₘ); 8,39 (m, 1 H, Hₐᵣₒₘ); 8,78 (m, 2 H, Hₐᵣₒₘ) ppm.
¹³C-NMR (CD₂Cl₂): 21,7; 31,3; 31,4; 35,1; 56,5; 113,0; 119,5; 121,9; 123,2; 123,3; 123,5; 125,2 (d, *J*_{CP}= 5,4 Hz); 126,3 (d, *J*_{CP}= 9,5 Hz); 128,5; 129,9; 132,0 (d, *J*_{CP}= 3,0 Hz); 132,6; 136,1; 136,2 (d, *J*_{CP}= 6,0 Hz); 143,9 (d, *J*_{CP}= 6,7 Hz); 149,3 (d, *J*_{CP}= 5,7 Hz); 152,1; 153,8 ppm.

### 9-(tert-Butyl)-6-((2-isopropyl-5-methylcyclohexyl)oxy)-4-methoxy-2-methyldibenzo[d,f][1,3,2]dioxaphosphepin.

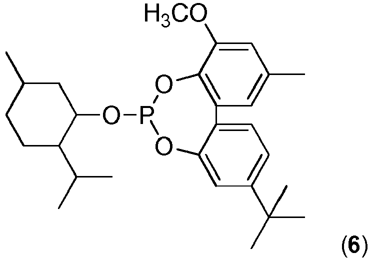

Eine gerührte Suspension von 4'-(*tert*-Butyl)-3-methoxy-5-methyl-[1,1'-biphenyl]-2,2'-diol (0,675 g; 2,36 mmol) in Toluol (16 ml) wurde mit Triethylamin (2,188 g; 21,62 mmol) versetzt und die erhaltene Mischung tropfenweise bei 0 °C zu einer Lösung von Dichlor((-)-menthyloxy)phosphin (0,606 g; 2,36 mmol) in Toluol (15 ml) gegeben. Die Reaktionsmischung wurde über Nacht gerührt und filtriert. Das Filtrat wurde im Vakuum zur Trockne eingeengt. Ausbeute: 0,965 g (2,051 mmol; 87 %).

Elementaranalyse (ber. für C₂₈H₃₉O₄P = 470,56 g/ mol) C 71,49 (71,46); H 8,59 (8,35); P 6,81 (6,58) %.
³¹P-NMR (CD₂Cl₂): 153,2; 153,4 ppm.
¹H-NMR (CD₂Cl₂): (m, 4 H); 0,97-1,02 (m, 6 H); 1,04-1,39 (m, 3 H); 1,41 (m, 9 H); 1,46-1,61 (m, 1 H); 1,73 (m, 2 H); 2,19-2,37 (m, 2 H); 2,43 (s, 3 H); 3,91 (s, 3 H); 4,15-4,28 (m, 1 H); 6,87 (m, 2 H, Hₐᵣₒₘ); 7,20-7,21 (m, 1 H, Hₐᵣₒₘ); 7,33-7,36 (m, 1 H, Hₐᵣₒₘ); 7,43-7,45 (m, 1 H, Hₐᵣₒₘ) ppm.

### 6-([1,1':3',1"-Terphenyl]-2'-yloxy)-2-isopropyl-8-methoxy-3,10-dimethyldibenzo[d,f][1,3,2]dioxaphosphepin.

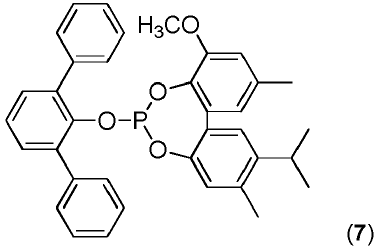

Eine Lösung von 5'-Isopropyl-3-methoxy-4',5-dimethyl-[1,1'-biphenyl]-2,2'-diol (0,478 g; 1,670 mmol) in Toluol (12 ml) wurde mit Triethylamin (1,548 g; 15,296 mmol) versetzt. Die Mischung wurde auf 0 °C gekühlt und eine Lösung von 2,6-Diphenylphenoxydichlorphosphin (0,580 g; 1,670 mmol) in Toluol (3 ml) tropfenweise zugegeben. Die Reaktionsmischung wurde über Nacht gerührt und dann filtriert. Das Filtrat wurde im Vakuum bis zur Trockne eingeengt, der erhaltene Feststoff bei 50°C getrocknet und durch Säulenchromatografie gereinigt (Dichlormethan, R*_{f}* = 0,8). Ausbeute: 0,642 g (1,144 mmol; 69 %).

Elementaranalyse (ber. für C₃₆H₃₃O₄P = 560,63 g/ mol) C 77,07 (77,13); H 5,73 (5,93); P 5,56 (5,52) %.
³¹P-NMR (CD₂Cl₂): 143,7 ppm.
¹H-NMR (CD₂Cl₂): (m, 6 H); 2,32 (m, 3 H); 2,40 (m, 3 H); 3,13-3,23 (m, 1 H); 3,76 (s, 3 H); 6,01 (m, 1 H, Hₐᵣₒₘ); 6,77 (m, 2 H, Hₐᵣₒₘ); 7,24 (m, 1 H, Hₐᵣₒₘ); 7,35-7,39 (m, 1 H, Hₐᵣₒₘ); 7,41-7,51 (m, 8 H, Hₐᵣₒₘ); 7,60-7,65 (m, 4 H, Hₐᵣₒₘ) ppm.
¹³C-NMR (CD₂Cl₂): 19,3; 21,6; 23,5; 23,6; 29,3; 56,1; 112,7; 121,3; 123,2; 125,0; 126,0; 127,8; 128,6; 129,2; 129,8; 130,7; 131,2; 133,0 (d, *J*_{CP}= 3,9 Hz); 134,9; 135,6; 136,5; 136,7; 139,0; 143,8; 146,6 (d, *J*_{CP}=5,4 Hz); 147,4 (d, *J*_{CP}=8,0 Hz); 152,0 (d, *J*_{CP}= 2,2 Hz) ppm.

### 6-(Anthracen-9-yloxy)-2-isopropyl-8-methoxy-3,10-dimethyldibenzo[d,f][1,3,2]dioxaphosphepin.

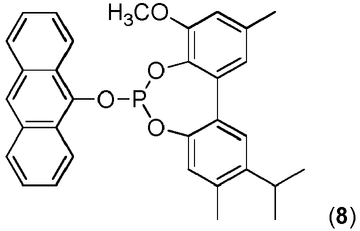

Eine Lösung von 5'-Isopropyl-3-methoxy-4',5-dimethyl-[1,1'-biphenyl]-2,2'-diol (0,359 g; 1,252 mmol) in Toluol (7 ml) wurde mit Triethylamin (1,161 g; 11,473 mmol) versetzt, auf 0 °C gekühlt und zu dieser Mischung eine Lösung von (Anthracen-9-yloxy)dichlorophosphin (0,370 g; 1,252 mmol) in Toluol (9 ml) tropfenweise zugegeben. Die Reaktionsmischung wurde über Nacht gerührt und dann filtriert. Das Filtrat wurde im Vakuum bis zur Trockne eingeengt. Ausbeute: 0,594 g (1,168 mmol; 93 %). Elementaranalyse (ber. für C₃₂H₂₉O₄P = 508,55 g/ mol) C 75,46 (75,58); H 5,90 (5,75); P 6,09 (6,09) %.
³¹P-NMR (CD₂Cl₂): 148,5 ppm.
¹H-NMR (CD₂Cl₂): 38 (d, ²*J*_{HH} = 6,8 Hz, 3 H); 1,39 (d, ²*J*_{CP} = 6,8 Hz, 3 H); 2,46 (s, 3 H); 2,52 (s, 3 H); 3,22-3,32 (m, 1 H); 3,99 (s, 3 H); 6,95 (m, 1 H, Hₐᵣₒₘ); 7,07 (m, 1 H, Hₐᵣₒₘ); 7,20 (m, 1 H, Hₐᵣₒₘ); 7,52 (s, 1 H, Hₐᵣₒₘ); 7,57-7,68 (m, 4 H, Hₐᵣₒₘ); 8,10 (m, 2 H, Hₐᵣₒₘ); 8,39 (m, 1 H, Hₐᵣₒₘ); 8,79 (m, 2 H, Hₐᵣₒₘ) ppm.
¹³C-NMR (CD₂Cl₂): 19,3; 21,7; 23,6; 23,6; 29,5; 56,5; 113,0; 121,9; 123,4; 123,5; 123,8; 125,2; (d, *J*_{CP}= 3,3 Hz); 125,7; 126,3 (d, *J*_{CP}= 3,3 Hz); 126,7; 128,6 (d, *J*_{CP}= 8,7 Hz); 129,4; 132,5 (d, *J*_{CP}= 3,6 Hz); 132,6; 136,1; 136,2 (d, *J*_{CP}= 6,2 Hz); 137,5; 138,4; 143,9 (d, *J*_{CP}= 6,5 Hz); 144,8; 146,9 (d, *J*_{CP}= 5,9 Hz); 152,1 ppm.

### 2-Isopropyl-6-((2-isopropyl-5-methylcyclohexyl)oxy)-8-methoxy-3,10-dimethyldibenzo[d,f][1,3,2]dioxaphosphepin.

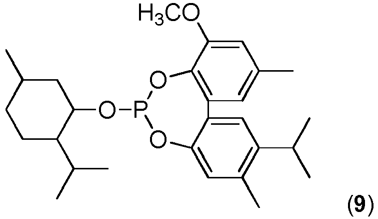

Eine Lösung von 5'-Isopropyl-3-methoxy-4',5-dimethyl-[1,1'-biphenyl]-2,2'-diol (0,664 g; 2,32 mmol) in Toluol (16 ml) wurde mit Triethylamin (2,152 g; 21,27 mmol) versetzt und die erhaltene Mischung tropfenweise bei 0 °C zu einer Lösung von Dichlor((-)-menthyloxy)phosphin (0,596 g; 2,32 mmol) in Toluol (15 ml) gegeben. Die Reaktionsmischung wurde über Nacht gerührt und filtriert. Das Filtrat wurde im Vakuum zur Trockne eingeengt. Ausbeute: 0,944 g (2,006 mmol; 87 %).

Elementaranalyse (ber. für C₂₈H₃₉O₄P = 470,56 g/ mol) C 71,28 (71,46); H 8,36 (8,35); P 6,72 (6,58) %.
³¹P-NMR (CD₂Cl₂): 152,4; 153,7 ppm.
¹H-NMR (CD₂Cl₂): (m, 4 H); 0,97-1,02 (m, 6 H); 1,06-1,29 (m, 2 H); 1,32 (m, 6 H); 1,35-1,46 (m, 1 H); 1,46-1,60 (m, 1 H); 1,73 (m, 2 H); 2,19-2,37 (m, 2 H); 2,40-2,46 (m, 6 H); 3,21 (s, 1 H); 3,90 (s, 3 H); 4,20 (m, 1 H); 6,84 (s, 1 H, Hₐᵣₒₘ); 6,90 (m, 1 H, Hₐᵣₒₘ); 6,96 (m, 1 H, Hₐᵣₒₘ); 7,35 (m, 1 H, Hₐᵣₒₘ) ppm.

### 6-([1,1':3',1"-Terphenyl]-2'-yloxy)-4-methoxy-2,10-dimethyldibenzo[d,f][1,3,2]dioxaphosphepin.

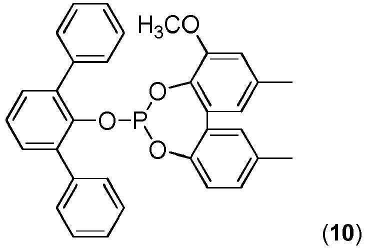

Eine gerührte Lösung von 3-Methoxy-5,5'-dimethyl-[1,1'-biphenyl]-2,2'-diol (0,465 g; 1,903 mmol) in Toluol (12 ml) wurde mit Triethylamin (1,764 g; 17,433 mmol) versetzt und auf 0°C gekühlt. Zu dieser Mischung wurde eine Lösung von 2,6-Diphenylphenoxydichlorphosphin (0,661 g; 1,903 mmol) in Toluol (3 ml) tropfenweise zugegeben. Die Reaktionsmischung wurde über Nacht gerührt und dann filtriert. Das Filtrat wurde im Vakuum bis zur Trockne eingeengt und der erhaltene Feststoff aus Hexan (20 ml) umkristallisiert. Ausbeute: 0,804 g (1,550 mmol; 82 %).

Elementaranalyse (ber. für C₃₃H₂₇O₄P = 518,55 g/ mol) C 76,38 (76,44); H 5,18 (5,25); P 5,97 (5,97) %.
³¹P-NMR (CD₂Cl₂): 144,9 ppm.
¹H-NMR (CD₂Cl₂): (m, 6 H); 3,75 (s, 3 H); 5,86 (m, 1 H, Hₐᵣₒₘ); 6,76 (m, 2 H, Hₐᵣₒₘ); 6,92 (m, 1 H, Hₐᵣₒₘ); 7,19 (m, 1 H, Hₐᵣₒₘ); 7,36 (m, 1 H, Hₐᵣₒₘ); 7,40-7,55 (m, 8 H, Hₐᵣₒₘ); 7,60-7,68 (m, 4 H, Hₐᵣₒₘ) ppm.
¹³C-NMR (CD₂Cl₂): 20,9; 21,6; 56,0; 112,9; 121,4; 121,8; 125,1; 127,7; 128,8; 129,9; 130,1; 130,7; 131,3; 132,7 (d, *J*_{CP}= 3,5 Hz); 134,4; 135,1; 135,7; 136,4 (d, *J*_{CP}= 4,4 Hz); 139,0; 146,4 (d, *J*_{CP}= 6,8 Hz); 147,6 (d, *J*_{CP}= 7,4Hz); 152,0 (d, *J*_{CP}=2,5 Hz) ppm.

### 6-(Anthracen-9-yloxy)-4-methoxy-2,10-dimethyldibenzo[d,f][1,3,2]dioxaphosphepin.

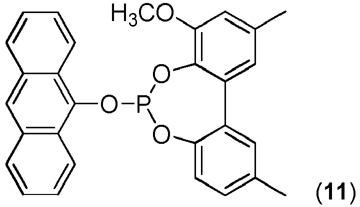

Eine gerührte Lösung von 3-Methoxy-5,5'-dimethyl-[1,1'-biphenyl]-2,2'-diol (0,655 g; 2,68 mmol) in Toluol (16 ml) wurde mit Triethylamin (2,487 g; 24,58 mmol) versetzt und auf 0°C gekühlt. Zu dieser Mischung wurde eine Lösung von (Anthracen-9-yloxy)dichlorophosphin (0,792 g; 2,683 mmol) in Toluol (18 ml) tropfenweise zugegeben. Die Reaktionsmischung wurde über Nacht gerührt und dann filtriert. Das Filtrat wurde im Vakuum bis zur Trockne eingeengt und der Rückstand 3 h bei 50°C / 0,1 mbar getrocknet. Ausbeute: 1,020 g (2,187 mmol; 82 %).

Elementaranalyse (ber. für C₂₉H₂₃O₄P = 466,47 g/ mol) C 74,58 (74,67); H 5,19 (4,97); P 6,78 (6,64) %.
³¹P-NMR (CD₂Cl₂): 148,6 ppm.
¹H-NMR (CD₂Cl₂): 2,51 (m, 6 H); 3,99 (s, 3 H); 6,96 (m, 1 H, Hₐᵣₒₘ); 7,06 (m, 1 H, Hₐᵣₒₘ); 7,32-7,37 (m, 2 H, Hₐᵣₒₘ); 7,48 (m, 1 H, Hₐᵣₒₘ); 7,57-7,69 (m, 4 H, Hₐᵣₒₘ); 8,10 (m, 2 H, Hₐᵣₒₘ); 8,39 (m, 1 H, Hₐᵣₒₘ); 8,79 (m, 2 H, Hₐᵣₒₘ) ppm.
¹³C-NMR (CD₂Cl₂): 21,1; 21,7; 56,4; 113,2; 122,0; 122,2; 123,3; 123,5; 125,1 (d, *J*_{CP}= 3,7 Hz); 126,2; 126,3; 128,5; 130,3; 130,9; 131,3 (d, *J*_{CP}= 3,2 Hz); 132,1 (d, *J*_{CP}= 3,3 Hz); 132,5 (d, *J*_{CP}= 2,0 Hz); 135,7; 136,1; 136,3 (d, *J*_{CP}= 5,8 Hz); 143,9 (d, *J*_{CP}= 7,1 Hz); 147,3 (d, *J*_{CP}= 5,4 Hz); 152,0 (d, *J*_{CP}= 2,0 Hz) ppm.

### 6-((2-Isopropyl-5-methylcyclohexyl)oxy)-4-methoxy-2,10-dimethyldibenzo[d,f][1,3,2]dioxaphosphepin.

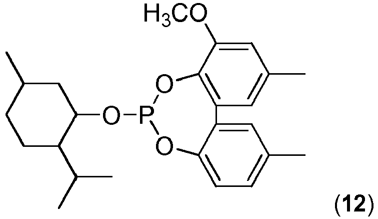

Eine gerührte Suspension von 3-Methoxy-5,5'-dimethyl-[1,1'-biphenyl]-2,2'-diol (0,56 g; 2,3 mmol) in Toluol (16 ml) wurde mit Triethylamin (2,132 g; 21,07 mmol) versetzt und die erhaltene Mischung tropfenweise bei 0 °C zu einer Lösung von Dichlor((-)-menthyloxy)phosphin (0,591 g; 2,3 mmol) in Toluol (15 ml) gegeben. Die Reaktionsmischung wurde über Nacht gerührt und filtriert. Das Filtrat wurde im Vakuum bis zur Trockne eingeengt. Ausbeute: 0,860 g (2,01 mmol; 87 %).

Elementaranalyse (ber. für C₂₅H₃₃O₄P = 428,48 g/ mol) C 70,26 (70,07); H 7,71 (7,76); P 7,09 (7,23) %.
³¹P-NMR (CD₂Cl₂): 152,9; 153,8 ppm.
¹H-NMR (CD₂Cl₂): (m, 4 H); 0,96-1,01 (m, 6 H); 1,04-1,46 (m, 3 H); 1,46-1,59 (m, 1 H); 1,72 (m, 2 H); 2,17-2,37 (m, 2 H); 2,43 (m, 6 H); 3,90 (s, 3 H); 4,20 (m, 1 H); 6,85 (m, 1 H, Hₐᵣₒₘ); 6,90 (m, 1 H, Hₐᵣₒₘ); 7,06 (m, 1 H, Hₐᵣₒₘ); 7,20 (m, 1 H, Hₐᵣₒₘ); 7,31 (m, 1 H, Hₐᵣₒₘ) ppm.

### 6-([1,1':3',1"-Terphenyl]-2'-yloxy)-4-methoxy-2,8,10-trimethyldibenzo[d,f][1,3,2]dioxaphosphepin.

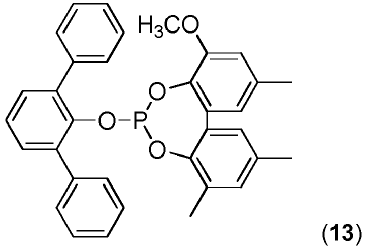

Eine Lösung von 3-Methoxy-3',5,5'-trimethyl-[1,1'-biphenyl]-2,2'-diol (0,492 g; 1,903 mmol) in Toluol (18 ml) wurde mit Triethylamin (1,764 g; 17,435 mmol) versetzt und auf 0 °C gekühlt. Zu dieser Mischung wurde eine Lösung von 2,6-Diphenylphenoxydichlorphosphin (0,727 g; 2,094 mmol) in Toluol (3 ml) tropfenweise zugegeben. Die Reaktionsmischung wurde über Nacht gerührt und dann filtriert. Das Filtrat wurde im Vakuum bis zur Trockne eingeengt, der erhaltene Feststoff bei 50 °C / 0,1 mbar getrocknet und durch Säulenchromatografie gereinigt (Hexan/Dichlormethan, 1:1, R*_{f}* = 0,5). Ausbeute: 0,946 g (1,776 mmol; 81 %). Elementaranalyse (ber. für C₃₄H₂₉O₄P = 532,57 g/ mol) C 76,56 (76,68); H 5,44 (5,49); P 5,95 (5,82) %.
³¹P-NMR (CD₂Cl₂): 145,6 ppm.
¹H-NMR (CD₂Cl₂): (m, 3 H); 2,42 (m, 6 H); 3,84 (s, 3 H); 6,77 (m, 2 H, Hₐᵣₒₘ); 7,03 (m, 2 H, Hₐᵣₒₘ); 7,29-7,34 (m, 5 H, Hₐᵣₒₘ); 7,34-7,37 (m, 1 H, Hₐᵣₒₘ); 7,37-7,40 (m, 1 H, Hₐᵣₒₘ); 7,44 (m, 1 H, Hₐᵣₒₘ); 7,47 (m, 1 H, Hₐᵣₒₘ); 7,55-7,62 (m, 4 H, Hₐᵣₒₘ) ppm.
¹³C-NMR (CD₂Cl₂): 17,0; 21,0; 21,6; 56,3; 112,8; 122,0; 125,0; 127,4; 128,2; 128,3; 129,3; 129,8; 130,3; 130,5; 130,8 (d, *J*_{CP}= 3,3 Hz); 131,0; 131,4; 132,7 (d, *J*_{CP}= 3,6 Hz); 134,2; 135,1; 136,0 (d, *J*_{CP}= 3,1 Hz); 138,9; 139,0; 146,2; 146,3 (d, *J*_{CP}= 6,9Hz); 151,7 (d, *J*_{CP}= 2,0 Hz) ppm.

### 6-(Anthracen-9-yloxy)-4-methoxy-2,8,10-trimethyldibenzo[d,f][1,3,2]dioxaphosphepin.

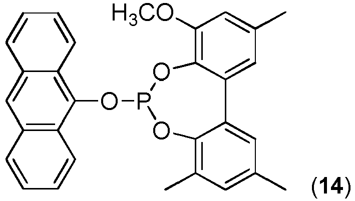

Eine Lösung von 3-Methoxy-3',5,5'-trimethyl-[1,1'-biphenyl]-2,2'-diol (0,409 g; 1,58 mmol) in Toluol (9 ml) wurde mit Triethylamin (2,468 g; 14,51 mmol) versetzt, auf 0 °C gekühlt und zu dieser Mischung eine Lösung von (Anthracen-9-yloxy)dichlorophosphin (0,467 g; 1,584 mmol) in Toluol (11 ml) tropfenweise zugegeben. Die Reaktionsmischung wurde über Nacht gerührt und dann filtriert. Das Filtrat wurde im Vakuum bis zur Trockne eingeengt und der erhaltene Rückstand aus Hexan (17 ml) umkristallisiert. Ausbeute: 0,511 g (1,063 mmol; 67 %). Elementaranalyse (ber. für C₃₀H₂₅O₄P = 480,50 g/ mol) C 74,53 (74,99); H 5,53 (5,24); P 6,48 (6,45) %.
³¹P-NMR (CD₂Cl₂): 147,7 ppm.
¹H-NMR (CD₂Cl₂): 2,46-2,50 (m, 9 H); 4,02 (s, 3 H); 6,96 (m, 1 H, Hₐᵣₒₘ); 7,05 (m, 1 H, Hₐᵣₒₘ); 7,20 (m, 1 H, Hₐᵣₒₘ); 7,30 (m, 1 H, Hₐᵣₒₘ); 7,56-7,68 (m, 4 H, Hₐᵣₒₘ); 8,10 (m, 2 H, Hₐᵣₒₘ); 8,40 (m, 1 H, Hₐᵣₒₘ); 8,88 (m, 2 H, Hₐᵣₒₘ) ppm.
¹³C-NMR (CD₂Cl₂): 17,0; 21,1; 21,7; 56,3; 113,0; 122,2; 123,4; 123,5; 125,3 (d, *J*_{CP}= 3,6 Hz); 125,7; 126,3 (d, *J*_{CP}= 5,5 Hz); 128,4; 128,5; 130,9; 131,6 (d, *J*_{CP}= 3,7 Hz); 132,0; 132,0; 132,5; 135,4; 135,8; 136,8 (d, *J*_{CP}= 8,0 Hz); 143,9 (d, *J*_{CP}= 7,4 Hz); 145,3 (d, *J*_{CP}= 4,0 Hz); 151,9 ppm.

### 6-((2-Isopropyl-5-methylcyclohexyl)oxy)-4-methoxy-2,8,10-trimethyldibenzo[d,f][1,3,2]dioxaphosphepin.

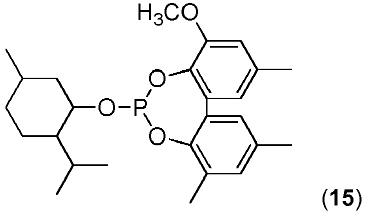

Eine Lösung von 3-Methoxy-3',5,5'-trimethyl-[1,1'-biphenyl]-2,2'-diol (0,594 g; 2,3 mmol) in Toluol (16 ml) wurde mit Triethylamin (2,132 g; 21,07 mmol) versetzt und tropfenweise bei 0°C zu einer Lösung von Dichlor((-)-menthyloxy)phosphin (0,591 g; 2,3 mmol) in Toluol (15 ml) gegeben. Die Reaktionsmischung wurde über Nacht gerührt und filtriert. Das Filtrat wurde im Vakuum bis zur Trockne eingeengt. Unmittelbar nach Lösen des Rückstandes in Dichlormethan (1,5 ml) erfolgte Kristallisation. Der erhaltene Feststoff wurde bei Raumtemperatur im Vakkum getrocknet. Ausbeute: 0.337 g (0.76 mmol, 33 %). Elementaranalyse (ber. für C₂₆H₃₅O₄P = 442,51 g/ mol): C 70,33 (70,57); H 8,05 (7,97); P 7,22 (7,00) %.
³¹P-NMR (CD₂Cl₂): 105,4; 153,6 ppm.
¹H-NMR (CD₂Cl₂): 0,86-0,93 (m, 4 H); 0,95-1,01 (m, 6 H); 1,03-1,19 (m, 1 H); 1,19-1,34 (m, 1 H); 1,34-1,46 (m, 1 H); 1,46-1,61 (m, 1 H); 1,67-1,77 (m, 2 H); 2,12-2,35 (m, 2 H); 2,35-2,44 (m, 9 H); 3,89 (s, 3 H); 4,20 (m, 1 H); 6,86 (m, 2 H, Hₐᵣₒₘ); 7,11 (m, 2 H, Hₐᵣₒₘ) ppm.

### 6-([1,1':3',1"-Terphenyl]-2'-yloxy)-8-methoxy-2,3,10-trimethyldibenzo[d,f][1,3,2]dioxaphosphepin.

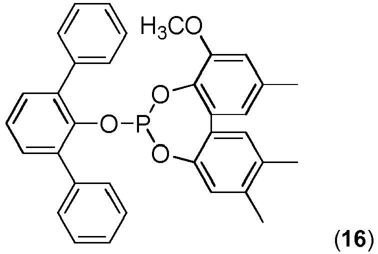

Eine gerührte Suspension von 3-Methoxy-4',5,5'-trimethyl-[1,1'-biphenyl]-2,2'-diol (0,492 g; 1,903 mmol) in Toluol (18 ml) wurde mit Triethylamin (1,764 g; 17,435 mmol) versetzt und auf 0 °C gekühlt. Zu dieser Mischung wurde eine Lösung von 2,6-Diphenylphenoxydichlorphosphin (0,727 g; 2,094 mmol) in Toluol (3 ml) tropfenweise zugegeben. Die Reaktionsmischung wurde über Nacht gerührt und dann filtriert. Das Filtrat wurde im Vakuum bis zur Trockne eingeengt, der erhaltene Feststoff bei 50 °C / 0,1 mbar getrocknet und durch Säulenchromatografie gereinigt (Hexan/Dichlormethan, 1:2, R*_{f}* = 0,3). Alternativ wurde die Substanz aus Hexan (23 ml) umkristallisiert. Ausbeute: 0,749 g (1,406 mmol; 74 %).

Elementaranalyse (ber. für C₃₄H₂₉O₄P = 532,57 g/ mol) C 76,56 (76,68); H 5,31 (5,49); P 5,90 (5,82) %.
³¹P-NMR (CD₂Cl₂): 144,0 ppm.
¹H-NMR (CD₂Cl₂): (m, 3 H); 2,33 (m, 3 H); 2,41 (m, 3 H); 3,77 (s, 3 H); 6,02 (m, 1 H, Hₐᵣₒₘ); 6,78 (m, 2 H, Hₐᵣₒₘ); 7,18 (m, 1 H, Hₐᵣₒₘ); 7,35-7,41 (m, 1 H, Hₐᵣₒₘ); 7,42-7,55 (m, 8 H, Hₐᵣₒₘ); 7,64-7,70 (m, 4 H, Hₐᵣₒₘ) ppm.
¹³C-NMR (CD₂Cl₂): 19,3; 19,9; 21,6; 56,1; 112,7; 121,4; 122,8; 125,1; 127,5 (d, *J*_{CP}= 2,7 Hz); 127,8; 128,6; 130,4; 130,5; 130,7; 131,3; 132,8 (d, *J*_{CP}= 3,8 Hz); 133,4; 135,1; 135,6; 136,6 (d, *J*_{CP}= 4,2 Hz); 138,1; 139,0; 146,6 (d, *J*_{CP}= 5,7 Hz); 147,8 (d, *J*_{CP}= 8,1 Hz); 152,0 (d, *J*_{CP}= 2,0 Hz) ppm.

### 6-(Anthracen-9-yloxy)-8-methoxy-2,3,10-trimethyldibenzo[d,f][1,3,2]dioxaphosphepin.

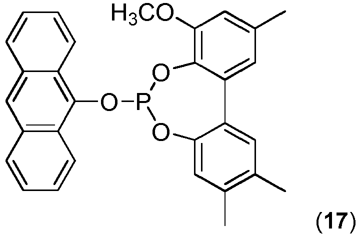

Eine Lösung von 3-Methoxy-4',5,5'-trimethyl-[1,1'-biphenyl]-2,2'-diol (0,518 g; 2,00 mmol) in Toluol (11 ml) wurde mit Triethylamin (1,857 g; 18,35 mmol) versetzt und auf 0 °C gekühlt. Zu dieser Mischung wurde eine Lösung von (Anthracen-9-yloxy)dichlorophosphin (0,591 g; 2,00 mmol) in Toluol (8 ml) tropfenweise zugegeben. Nach Zugabe von weiterem Toluol (30 ml) wurde die Reaktionsmischung über Nacht gerührt. Dann wurde die Mischung filtriert und das Lösungsmittel im Vakuum entfernt. Der erhaltene Rückstand wurde in Toluol (11 ml) aufgenommen, die resultierende Suspension leicht erhitzt und dann filtriert. Das Filtrat wurde im Vakuum bis zur Trockne eingeeengt und der erhaltene Feststoff bei 50 °C / 0.1 mbar getrocknet. Ausbeute: 0,489 g (1,018 mmol; 51 %). Elementaranalyse (ber. für C₃₀H₂₅O₄P = 480,50 g/ mol) C 74,77 (74,99); H 5,10 (5,24); P 6,47 (6,45) %.
³¹P-NMR (CD₂Cl₂): 148,2 ppm.
¹H-NMR (CD₂Cl₂): 2,38 (m, 6 H); 2,49 (m, 3 H); 3,98 (s, 3 H); 6,93 (m, 1 H, Hₐᵣₒₘ); 7,03 (m, 1 H, Hₐᵣₒₘ); 7,19 (m, 1 H, Hₐᵣₒₘ); 7,40 (m, 1 H, Hₐᵣₒₘ); 7,56-7,68 (m, 4 H, Hₐᵣₒₘ); 8,09 (m, 2 H, Hₐᵣₒₘ); 8,38 (m, 1 H, Hₐᵣₒₘ); 8,76 (m, 2 H, Hₐᵣₒₘ) ppm.
¹³C-NMR (CD₂Cl₂): 19,4; 19,8; 21,7; 56,4; 112,9; 121,8; 123,3; 123,3; 123,4; 125,1 (d, *J*_{CP}= 3,5 Hz); 125,6 (d, *J*_{CP}= 3,5 Hz); 126,1; 126,2; 128,4; 131,1; 132,1 (d, *J*_{CP}= 3,1 Hz); 132,5; 134,4; 136,0; 136,2 (d, *J*_{CP}= 6,0 Hz); 138,8; 143,9 (d, *J*_{CP}= 6,9 Hz); 147,2 (d, *J*_{CP}= 5,7 Hz); 152,0 ppm.

### 6-((2-Isopropyl-5-methylcyclohexyl)oxy)-8-methoxy-2,3,10-trimethyldibenzo[d,f][1,3,2]dioxaphosphepin.

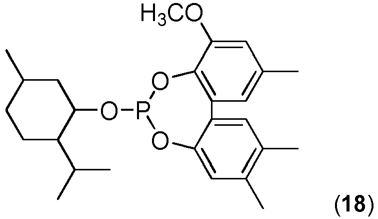

Eine gerührte Lösung von 3-Methoxy-4',5,5'-trimethyl-[1,1'-biphenyl]-2,2'-diol (0,592 g; 2,30 mmol) in Toluol (16 ml) wurde mit Triethylamin (2,127 g; 21,02 mmol) versetzt und die erhaltene Mischung tropfenweise bei 0 °C zu einer Lösung von Dichlor((-)-menthyloxy)phosphin (0,588 g; 2,30 mmol) in Toluol (15 ml) gegeben. Die Reaktionsmischung wurde über Nacht gerührt und dann filtriert. Das Filtrat wurde im Vakuum bis zur Trockne eingeengt. Ausbeute: 0,679 g (1,535 mmol; 67 %). Elementaranalyse (ber. für C₂₆H₃₅O₄P = 442,51 g/ mol) C 70,70 (70,57); H 8,32 (7,97) %. ³¹P-NMR (CD₂Cl₂): 152,5; 153,5 ppm.
¹H-NMR (CD₂Cl₂): 0,90 (m, 4 H); 0,96-1,02 (m, 6 H); 1,05-1,45 (m, 3 H); 1,46-1,59 (m, 1 H); 1,73 (m, 2 H); 2,17-2,32 (m, 2 H); 2,34 (m, 6 H); 2,43 (m, 3 H); 3,90 (s, 3 H); 4,13-4,25 (m, 1 H); 6,83 (s, 1 H, Hₐᵣₒₘ); 6,89 (m, 1 H, Hₐᵣₒₘ); 6,97 (m, 1 H, Hₐᵣₒₘ); 7,26 (m, 1 H, Hₐᵣₒₘ) ppm.

### 6-([1,1':3',1"-Terphenyl]-2'-yloxy)dibenzo[d,f][1,3,2]dioxaphosphepin

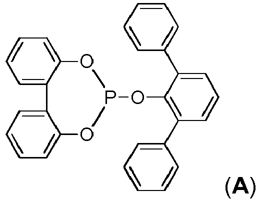

Eine Lösung von 2,6-Diphenylphenol (0,491 g; 1,99 mmol) in Toluol (9 ml) wurde mit Triethylamin (1,851 g; 18,293 mmol) versetzt und die erhaltene Mischung tropfenweise bei 0°C zu einer Lösung von 6-Chlorodibenzo[*d*,*f*][1,3,2]dioxaphosphepin (0,500 g; 1,99 mmol) in Toluol (9 ml) gegeben. Die Reaktionsmischung wurde über Nacht gerührt und dann filtriert. Das Filtrat wurde im Vakuum bis zur Trockne eingeengt und der erhaltene Rückstand aus heißem Toluol (3 ml) umkristallisiert. Ausbeute: 0,536 g (1,164 mmol; 58 %). Elementaranalyse (ber. für C₃₀H₂₁O₃P = 460,44 g/ mol) C 78,30 (78,25); H 4,72 (4,60) %. ³¹P-NMR (CD₂Cl₂): 146,0 ppm.
¹H-NMR (CD₂Cl₂₎: 6,12 (m, 2 H); 7,21 (m, 4 H); 7,35-7,40 (m, 3 H); 7,43-7,46 (m, 2 H); 7,52-7,57 (m, 6 H); 7,59-7,64 (m, 4 H) ppm.
¹³C-NMR(CD₂Cl₂): 122,5; 125,2; 125,5; 127,9; 128,9; 129,5; 129,8; 131,1; 131,2; 136,4; 136,5; 138,9; 146,5 (d, *J*_{CP}= 8,5 Hz); 149,0 (d, *J*_{CP}= 4,7 Hz) ppm.

### 6-([1,1':3',1"-Terphenyl]-2'-yloxy)-4,8-di-tert-butyl-2,10-dimethoxydibenzo-[d,f][1,3,2]dioxaphosphepin

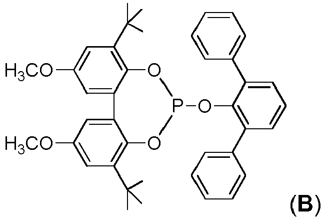

Eine Lösung von 2,6-Diphenylphenol (0,411 g; 1,65 mmol) in Toluol (8 ml) wurde mit Triethylamin (1,529 g; 15,11 mmol) versetzt und die erhaltene Mischung tropfenweise bei 0 °C zu einer Lösung von 4,8-Di-*tert*-butyl-6-chloro-2,10-dimethoxydibenzo-[*d*,*f*][1,3,2]dioxaphosphepin (0,697 g; 1,65 mmol) in Toluol (6 ml) gegeben. Die Reaktionsmischung wurde über Nacht bei Raumtemperatur und für 5 h bei 70°C gerührt. Dann wurde die Mischung filtriert und das Filtrat im Vakuum bis zur Trockne eingeengt. Der erhaltene Rückstand wurde aus Hexan (4 ml) umkristallisiert. Ausbeute: 0,417 g (0,659 mmol; 40 %). Elementaranalyse (ber. für C₄₀H₄₁O₅P = 632,70 g/ mol) C 75,95 (75,93); H 6,52 (6,53); P 5,01 (5,00) %.
³¹P-NMR (CD₂Cl₂): 140,9 ppm.
¹H-NMR (CD₂Cl₂): 1,37 (s, 18 H); 3,84 (s, 6 H); 6,51 (d, ⁵*J*_{HH}= 3,1 Hz, 2 H, Hₐᵣₒₘ); 6,89-7,95 (m, br, 15 H, Hₐᵣₒₘ) ppm.
¹³C-NMR (CD₂Cl₂): 31,1; 35,5; 55,9; 113,0; 114,5; 125,2; 126,5-131,0 (broad, overlapping signals); 133,8 (d, *J*_{CP}= 4,0 Hz); 141,5 (d, *J*_{CP}= 6,3 Hz); 142,6; 144,8; 156,1 ppm.

### 6-(Anthracen-9-yloxy)-2,4,8,10-tetra-tert-butyldibenzo[d,f][1,3,2]dioxaphosphepin

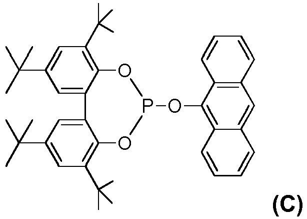

Eine gerührte Suspension von Anthracen-9-ol (0,281 g; 1,45 mmol) in Toluol (7 ml) wurde mit Triethylamin (1,344 g; 13,28 mmol) versetzt und die erhaltene Mischung tropfenweise bei 0°C zu einer Lösung von 2,4,8,10-Tetra-*tert*-butyl-6-chlorodibenzo[*d*,*f*][1,3,2]dioxaphosphepin (0,724 g; 1,52 mmol) in Toluol (6 ml) gegeben. Die Reaktionsmischung wurde über Nacht gerührt und filtriert. Das Filtrat wurde im Vakuum bis zur Trockne eingeengt und der erhaltene Rückstand aus Hexan (4 ml) umkristallisiert. Ausbeute: 0,559 g (0,883 mmol; 61 %). Elementaranalyse (ber. für C₄₂H₄₉O₃P = 632,78 g/ mol) C 79,83 (79,71); H 7,61 (7,81); P 5,01 (4,89) %.
³¹P-NMR (CD₂Cl₂): 140,7 ppm.
¹H-NMR (CD₂Cl₂): 1,41 (s, 18 H, C(C*H*₃)₃); 1,50 (s, 18 H, C(C*H*₃)₃); 7,40 (d, ⁵*J*_{HH}= 2,4 Hz, 2 H, Hₐᵣₒₘ); 7,43-7,56 (m, 4 H, Hₐᵣₒₘ); 7,60 (d, ⁵*J*_{HH}= 2,4 Hz, 2 H, Hₐᵣₒₘ); 8,05 (m, 2 H, Hₐᵣₒₘ); 8,34 (s, 1 H, Hₐᵣₒₘ); 8,38 (m, 2 H, Hₐᵣₒₘ) ppm.
¹³C-NMR (CD₂Cl₂): 31,2; 31,2; 31,8; 35,1; 35,7; 123,1; 123,7 (d, *J*_{CP}= 5,3 Hz); 124,8; 125,0; 125,8; 126,0; 127,2; 128,4; 132,6; 133,3 (d, *J*_{CP}= 3,7 Hz); 141,0; 143,5; 145,8 (d, *J*_{CP}= 6,2 Hz); 147,7 ppm.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Die als Substrat eingesetzten Substrate 1-Octen (Aldrich), *cis*/*trans*-2-Penten (Aldrich) und n-Octene (Oxeno GmbH, Octenisomerengemisch aus 1-Octen: 3,3 %; *cis*+*trans-*2-Octen: 48,5 %; *cis*+*trans-*3-Octen: 29,2%; *cis*+*trans-*Octen-4: 16,4 %; gerüstisomere Octene: 2,6 %) wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse, s.u.) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). Der Autoklav wurde bei einem Gesamtgasdruck (Synthesegas: Linde; H₂ (99,999%) : CO (99,997%) = 1:1) von a) 42 bar für einen Enddruck von 50 bar bzw. b) 12 bar für den

Enddruck von 20 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar bzw. b) 19,5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. Bronkhorst, NL) über 4h geführt. Simultan zum Reaktionsverlauf wurde in einem Zeittakt von 3 sec. der momentan anliegende Gasfluss bestimmt (Flowmeter der Fa. Bronkhorst). Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

In Tabelle 1 sind die Ergebnisse der Katalyseversuche aufgelistet.
Die erfindungsgemäßen Verbindungen sind hierbei mit * gekennzeichnet.

Als Vergleichsliganden wurden die Liganden **A**, **B** und **C** eingesetzt.
Solvens: Toluol
Ausb. = Ausbeute
p = Druck in [bar]
T = Temperatur in [°C]
t = Zeit in [h]
[Rh] = Rhodiumkonzentration in [ppm]
L/Rh = Verhältnis von Ligand zum Rhodium

**Tabelle 1: n-Octene**

| **Ligand** | **p (bar)** | **T(°C)** | **t(h)** | **[Rh] (ppm)** | **L/Rh** | **Sol.** | **Ausb. (%)** |
|---|---|---|---|---|---|---|---|
| **4*** | **20** | **120** | **4** | **100** | **5** | **Tol** | **88** |
| **5*** | **50** | **120** | **4** | **100** | **5** | **Tol** | **100** |
| **6*** | **20** | **120** | **4** | **100** | **5** | **Tol** | **92** |
| **7*** | **20** | **120** | **4** | **100** | **5** | **Tol** | **95** |
| **8*** | **50** | **120** | **4** | **100** | **5** | **Tol** | **99** |
| **9*** | **50** | **120** | **4** | **100** | **5** | **Tol** | **95** |
| **9*** | **20** | **120** | **4** | **100** | **5** | **Tol** | **91** |
| **10*** | **20** | **120** | **4** | **100** | **5** | **Tol** | **93** |
| **10*** | **50** | **120** | **4** | **100** | **5** | **Tol** | **95** |
| **11*** | **50** | **120** | **4** | **100** | **5** | **Tol** | **99** |
| **11*** | **20** | **120** | **4** | **100** | **5** | **Tol** | **98** |
| **11*** | **50** | **120** | **4** | **40** | **5** | **Tol** | **100** |
| **11*** | **50** | **120** | **4** | **60** | **5** | **Tol** | **100** |
| **12*** | **50** | **120** | **4** | **100** | **5** | **Tol** | **92** |
| **13*** | **20** | **120** | **4** | **100** | **5** | **Tol** | **91** |
| **14*** | **50** | **120** | **4** | **100** | **5** | **Tol** | **99** |
| **14*** | **20** | **120** | **4** | **100** | **5** | **Tol** | **98** |
| **14*** | **50** | **120** | **4** | **40** | **5** | **Tol** | **99** |
| **14*** | **50** | **120** | **4** | **60** | **5** | **Tol** | **100** |
| **15*** | **50** | **120** | **4** | **100** | **5** | **Tol** | **99** |
| **16*** | **20** | **120** | **4** | **100** | **5** | **Tol** | **97** |
| **17*** | **50** | **120** | **4** | **100** | **5** | **Tol** | **99** |
| **18*** | **50** | **120** | **4** | **100** | **5** | **Tol** | **96** |
| **A** | **50** | **120** | **4** | **100** | **5** | **Tol** | **91** |
| **B** | **20** | **120** | **4** | **100** | **5** | **Tol** | **29** |
| **C** | **20** | **120** | **4** | **100** | **5** | **Tol** | **86** |

Wie der Tabelle 1 zu entnehmen ist, zeichnen sich die erfindungsgemäßen Verbindungen in der Hydroformylierung von Olefinmischungen, konkret den n-Octenen, die sowohl interne als auch endständige Olefine enthalten, durch sehr gute Ausbeuten aus. Alle erfindungsgemäßen Verbindungen haben eine Ausbeute, welche besser ist, als jene, welche mit den Vergleichsliganden **A** (50 bar), **B** (20 bar) und **C** (20 bar) bei den entsprechenden Drücken erzielt werden konnten. So lag die Ausbeute bei einem Druck von 50 bar immer über dem Wert von 91 %, welcher mit dem Vergleichsligand **A** erzielt wurde.

Insbesondere bei einem Druck von 20 bar zeichnen sich die erfindungsgemäßen Verbindungen durch deutlich bessere Ausbeuten als die Vergleichsliganden B und **C** aus.

Wie die Versuchsergebnisse zeigen, wird die gestellte Aufgabe durch die erfindungsgemäßen Verbindungen gelöst.

Es ist somit erstmalig gelungen, Monophosphite zu generieren, die einen unsymmetrischen Biaryl-Baustein enthalten und sehr gute Hydroformylierungseigenschaften aufweisen. Dies konnte mit einer Vielzahl von Beispielen belegt werden. Solche konkreten Strukturen und derartige Liganden waren bis dato gänzlich unbekannt und nicht zugänglich.
Diese Monophosphite weisen eine neuartige Asymmetrie auf. Das besondere hierbei ist die Asymmetrie innerhalb des Biaryl-Bausteins, die zu unsymmetrischen Monophosphiten führt. Diese unsymmetrischen Monophosphite sind strukturell somit gänzlich von denen im Stand der Technik beschriebenen unsymmetrischen Monophoshiten verschieden, bei denen die Asymmetrie durch den am dritten Sauerstoff gebundenen Rest (X) bedingt wird.

## Patentansprüche

1. Verbindung, welche die allgemeine Struktur **I** aufweist: 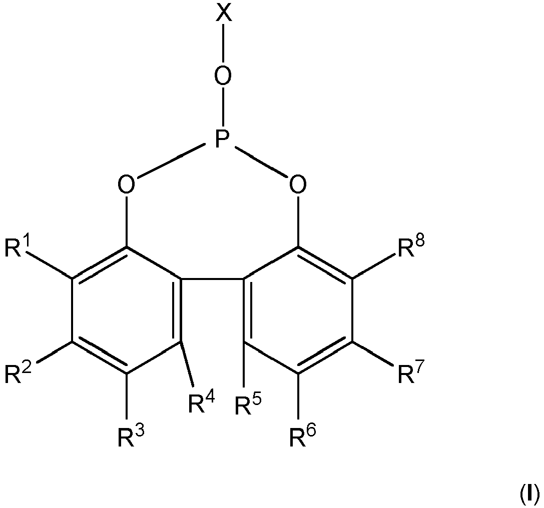 wobei
R¹, R², R³, R⁴, R⁵, R⁶, R⁷, R⁸ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl,, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
X ausgewählt ist aus:
- (C₁-C₁₂)-Alkyl, -(C₆-C₂₀)-Aryl, -(C₃-C₁₂)-Cycloalkyl, -(C₃-C₁₂)-Heterocycloalkyl,
und die beiden Reste mindestens eines der vier folgenden Restepaare nicht für den gleichen Rest stehen: R¹ und R⁸, R² und R⁷, R³ und R⁶, R⁴ und R⁵;
und die Reste R³ und R⁴ sind nicht über eine Kohlenstoffkette miteinander verknüpft;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen, die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen, diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl,-(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H;-SO₃Na, -NO₂, -CN, -NH₂, -N[(C₁-C₁₂)-Alkyl]₂.

2. Verbindung nach Anspruch 1,
wobei R¹, R², R³, R⁴, R⁵, R⁶, R⁷, R⁸ ausgewählt sind aus:
- H, -(C₁-C₁₂)-Alkyl, -O-(C₁-C₁₂)-Alkyl, -O-(C₆-C₂₀)-Aryl, -S-Alkyl, -S-Aryl.

3. Verbindung nach einem der Ansprüche 1 bis 2,
wobei X ausgewählt ist aus:
- (C₁-C₁₂)-Alkyl, -(C₆-C₂₀)-Aryl, -(C₃-C₁₂)-Cycloalkyl.

4. Verbindung nach einem der Ansprüche 1 bis 3,
wobei R¹ und R⁸ nicht für den gleichen Rest stehen.

5. Verbindung nach einem der Ansprüche 1 bis 4,
wobei X für den folgenden Rest steht:

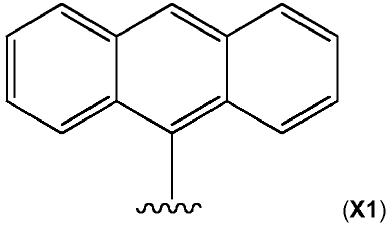

6. Verbindung nach einem der Ansprüche 1 bis 4,
wobei X für den folgenden Rest steht:

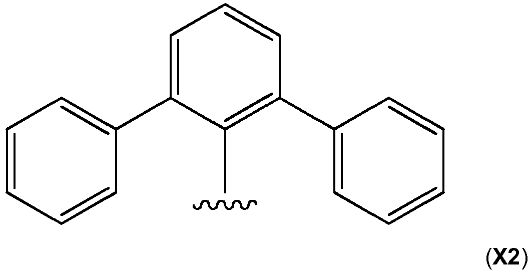

7. Komplex umfassend:
- eine Verbindung nach einem der Ansprüche 1 bis 6,
- ein Metallatom ausgewählt aus: Rh, Ru, Co, Ir.

8. Verwendung einer Verbindung nach einem der Ansprüche 1 bis 6,
zur Katalyse einer Hydroformylierungsreaktion.

9. Verfahren umfassend die Verfahrensschritte:
a) Vorlegen eines Olefins,
b) Zugabe eines Komplexes nach Anspruch 7,
oder einer Verbindung nach einem der Ansprüche 1 bis 6 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having the general structure I: 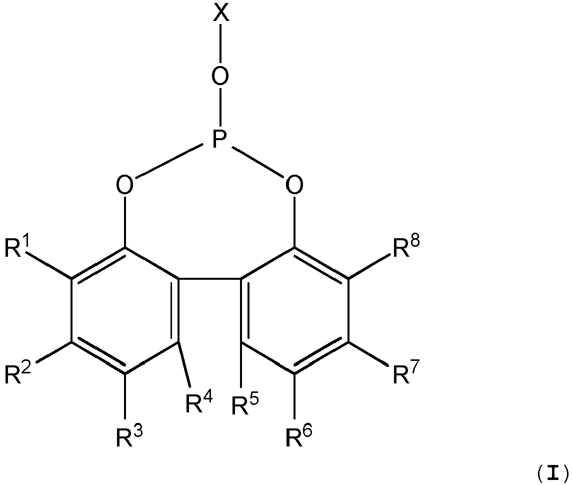 where
R¹, R², R³, R⁴, R⁵, R⁶, R⁷, R⁸ are selected from:
- H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
X is selected from:
- (C₁-C₁₂)-alkyl, -(C₆-C₂₀)-aryl, -(C₃-C₁₂)-cycloalkyl, -(C₃-C₁₂)-heterocycloalkyl,
and the two radicals in at least one of the four following radical pairs are not the same radical: R¹ and R⁸, R² and R⁷, R³ and R⁶, R⁴ and R⁵;
and the R³ and R⁴ radicals are not joined to one another via a carbon chain;
where the alkyl and aryl groups mentioned may be substituted as follows:
substituted -(C₁-C₁₂)-alkyl groups and substituted -(C₁-C₁₂)-alkoxy groups may have one or more substituents, depending on their chain length, the substituents are each independently selected from -(C₃-C₁₂)-cycloalkyl, -(C₃-C₁₂)-heterocycloalkyl, -(C₆-C₂₀)-aryl, fluorine, chlorine, cyano, formyl, acyl or alkoxycarbonyl; substituted -(C₆-C₂₀)-aryl groups and -(C₆-C₂₀)-aryl-(C₆-C₂₀)-aryl groups may have one or more substituents, depending on the ring size, and these substituents are each independently selected from -H, - (C₁-C₁₂)-alkyl, -O-(C₁-C₁₂)-alkyl, -O-(C₆-C₂₀)-aryl, -(C₆-C₂₀)-aryl, -halogen, -COO-(C₁-C₁₂)-alkyl, -CONH-(C₁-C₁₂)-alkyl, -(C₆-C₂₀)-aryl-CON[(C₁-C₁₂)-alkyl]₂, -CO-(C₁-C₁₂)-alkyl, -CO-(C₆-C₂₀)-aryl, -COOH, -OH, -SO₃H; -SO₃Na, -NO₂, -CN, -NH₂, -N[(C₁-C₁₂)-alkyl]₂.

2. Compound according to Claim 1,
where R¹, R², R³, R⁴, R⁵, R⁶, R⁷, R⁸ are selected from:
- H, -(C₁-C₁₂)-alkyl, -O-(C₁-C₁₂)-alkyl, -O-(C₆-C₂₀)-aryl, -S-alkyl, -S-aryl.

3. Compound according to either of Claims 1 and 2, where X is selected from:
- (C₁-C₁₂)-alkyl, -(C₆-C₂₀)-aryl, -(C₃-C₁₂)-cycloalkyl.

4. Compound according to any of Claims 1 to 3,
where R¹ and R⁸ are not the same radical.

5. Compound according to any of Claims 1 to 4,
where X is the following radical:

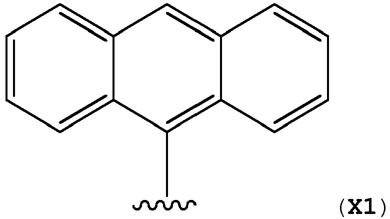

6. Compound according to any of Claims 1 to 4, where X is the following radical:

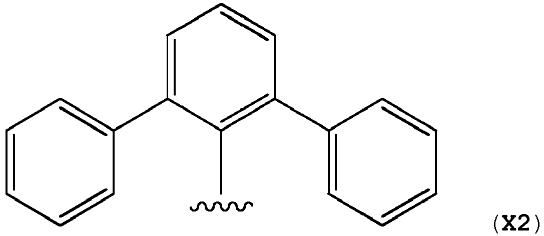

7. Complex comprising:
- a compound according to any of Claims 1 to 6,
- a metal atom selected from: Rh, Ru, Co, Ir.

8. Use of a compound according to any of Claims 1 to 6 for catalysing a hydroformylation reaction.

9. Process comprising the following process steps:
a) initially charging an olefin,
b) adding a complex according to Claim 7,
or a compound according to any of Claims 1 to 6 and a substance having a metal atom selected from: Rh, Ru, Co, Ir,
c) feeding in H₂ and CO,
d) heating the reaction mixture, with conversion of the olefin to an aldehyde.

## Revendications

1. Composé qui présente la structure générale I : 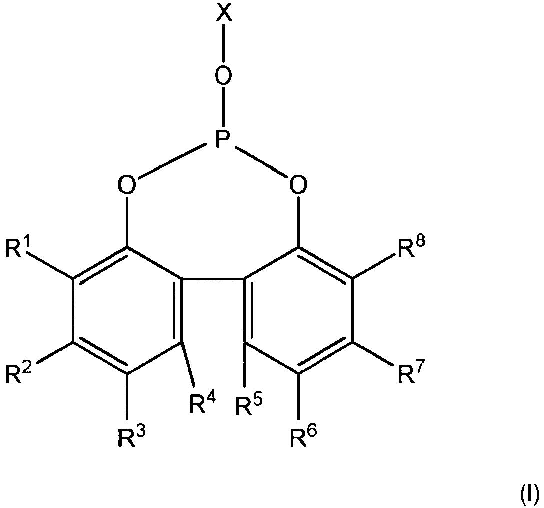 dans laquelle
R¹, R², R³, R⁴, R⁵, R⁶, R⁷, R⁸ sont choisis parmi :
H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogène, COO-(C₁-C₁₂)-alkyle, -CONH-(C₁-C₁₂)-alkyle, -CO-(C₁-C₁₂)-alkyle, -CO-(C₆-C₂₀)-aryle, -COOH, -OH, -SO₃H, -CN, -NH₂, -N[(C₁-C₁₂)-alkyle]₂ ;
X est choisi parmi :
- (C₁-C₁₂)-alkyle, -(C₆-C20)-aryle, -(C₃-C₁₂)-cycloalkyle, -(C₃-C₁₂)-hétérocycloalkyle,
et les deux radicaux d'au moins une des quatre paires de radicaux suivantes ne représentent pas le même radical :
R¹ et R⁸, R² et R⁷, R³ et R⁶, R⁴ et R⁵ ;
et les radicaux R³ et R⁴ ne sont pas liés l'un à l'autre via une chaîne carbonée ;
les groupes alkyle et aryle mentionnés pouvant être substitués comme suit :
les groupes -(C₁-C₁₂)-alkyle substitués et les groupes -(C₁-C₁₂)-alcoxy substitués peuvent présenter, en fonction de leur longueur de chaîne, un ou plusieurs substituants, les substituants sont choisis, indépendamment les uns des autres, parmi -(C₃-C₁₂)-cycloalkyle, -(C₃-C₁₂)-hétérocycloalkyle, -(C₆-C₂₀)-aryle, fluor, chlore, cyano, formyle, acyle ou alcoxycarbonyle ;
les groupes -(C₆-C₂₀)-aryle substitués et les groupes -(C₆-C₂₀)-aryl-(C₆-C₂₀)-aryle substitués peuvent présenter, en fonction de leur dimension de cycle, un ou plusieurs substituants, ces substituants sont choisis, indépendamment les uns des autres, parmi -H, -(C₁-C₁₂)-alkyle, -O-(C₁-C₁₂)-alkyle, -O-(C₆-C₂₀)-aryle, -(C₆-C₂₀)-aryle, -halogène, -COO-(C₁-C₁₂)-alkyle, -CONH-(C₁-C₁₂)-alkyle, -(C₆-C₂₀)-aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, -N[(C₁-C₁₂)-alkyle]₂.

2. Composé selon la revendication 1,
R¹, R², R³, R⁴, R⁵, R⁶, R⁷, R⁸ étant choisis parmi :
- H, -(C₁-C₁₂)-alkyle, -O-(C₁-C₁₂)-alkyle, -O-(C₆-C₂₀)-aryle, -S-alkyle, -S-aryle.

3. Composé selon l'une quelconque des revendications 1 à 2,
X étant choisi parmi :
- (C₁-C₁₂)-alkyle, -(C₆-C₂₀)-aryle, -(C₃-C₁₂)-cycloalkyle.

4. Composé selon l'une quelconque des revendications 1 à 3,
R¹ et R⁸ ne représentant pas le même radical.

5. Composé selon l'une quelconque des revendications 1 à 4,
X représentant le radical suivant :

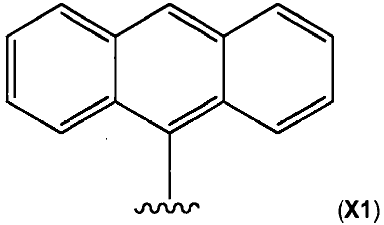

6. Composé selon l'une quelconque des revendications 1 à 4,
X représentant le radical suivant :

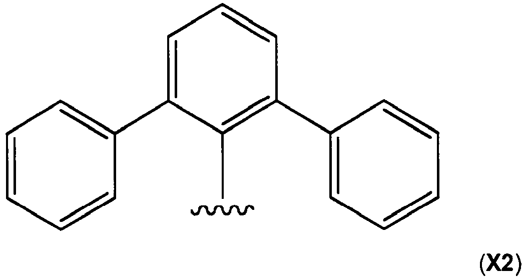

7. Complexe, comprenant :
- un composé selon l'une quelconque des revendications 1 à 6,
- un atome métallique choisi parmi : Rh, Ru, Co, Ir.

8. Utilisation d'un composé selon l'une quelconque des revendications 1 à 6 pour la catalyse d'une réaction d'hydroformylation.

9. Procédé comprenant les étapes de procédé :
a) disposition préalable d'une oléfine,
b) addition d'un complexe selon la revendication 7,
ou d'un composé selon l'une quelconque des revendications 1 à 6 et d'une substance qui présente un atome métallique choisi parmi : Rh, Ru, Co, Ir,
c) introduction de H₂ et de CO,
d) chauffage du mélange réactionnel, l'oléfine étant transformée en aldéhyde.
